# EUROPÄISCHE PATENTSCHRIFT

(11) **EP 3 851 893 B1**
(45) Veröffentlichungstag und Bekanntmachung des Hinweises auf die Patenterteilung: **11.05.2022**
(21) Anmeldenummer: 21150897.3
(22) Anmeldetag: 11.01.2021
(51) Int. Cl.: G02B 7/00, A61B 90/20, G02B 21/22, G02B 26/00, G02B 21/16, G02B 21/24, A61B 1/00

(54) **FILTERWECHSELVORRICHTUNG FÜR EIN OPTISCHES BEOBACHTUNGSINSTRUMENT MIT ZWEI STRAHLENGÄNGEN, OPTISCHES BEOBACHTUNGSINSTRUMENT UND VERFAHREN ZUM WECHSELN EINES FILTERS EINES OPTISCHEN BEOBACHTUNGSINSTRUMENTS**
FILTER CHANGING DEVICE FOR AN OPTICAL OBSERVATION INSTRUMENT WITH TWO BEAM PATHS, OPTICAL OBSERVATION INSTRUMENT AND METHOD FOR CHANGING A FILTER OF AN OPTICAL OBSERVATION INSTRUMENT
DISPOSITIF DE CHANGEMENT DE FILTRE POUR UN INSTRUMENT OPTIQUE D'OBSERVATION À DEUX CHEMINS DE FAISCEAU, INSTRUMENT OPTIQUE D'OBSERVATION ET PROCÉDÉ DE CHANGEMENT D'UN FILTRE D'UN INSTRUMENT OPTIQUE D'OBSERVATION

(30) Priorität: 14.01.2020 DE 102020100676
(43) Veröffentlichungstag der Anmeldung: 21.07.2021
(73) Patentinhaber: Karl Storz SE & Co. KG, 78532 Tuttlingen (DE)
(72) Erfinder: Forster, Jonas, 78532 Tuttlingen (DE)

(56) Entgegenhaltungen:
- EP-A1- 1 538 471
- CN-B- 106 200 213
- DE-A1- 10 336 890
- DE-A1-102006 004 232
- DE-A1-102009 011 681

## Beschreibung

Die vorliegende Erfindung betrifft eine Filterwechselvorrichtung für ein optisches Beobachtungsinstrument mit zwei Strahlengängen, insbesondere für ein stereoskopisches Beobachtungsinstrument, etwa ein Stereo-Videoendoskop, ein Stereo-Exoskop oder ein Stereo-Operationsmikroskop. Weiter betrifft die Erfindung ein derartiges optisches Beobachtungsinstrument sowie ein Verfahren zum Wechseln eines Filters eines optischen Beobachtungsinstruments mit zwei Strahlengängen.

Zur Beobachtung eines Operationsfelds bei einem chirurgischen Eingriff an einem menschlichen oder tierischen Körper sind optische Beobachtungsinstrumente bekannt, die einem Operateur sowie ggf. weiteren Personen eine genaue oder vergrößerte Betrachtung des Operationsfelds bzw. eines Objektfelds an dem Körper gestatten. Derartige optische Beobachtungsinstrumente können beispielsweise als Operationsmikroskop oder als Exoskop ausgebildet sein. Ebenso sind als Endoskope ausgebildete optische Beobachtungsinstrumente zur Beobachtung eines Objektfelds in einem körperinneren Hohlraum bekannt.

Bei der Durchführung einer chirurgischen Operation ist eine räumliche Wahrnehmung des Objektfelds für den operierenden Chirurgen hilfreich. Es ist bekannt, mit einer stereoskopischen Optik, bei der zwei Bilder des Objektfelds aus etwas unterschiedlicher Perspektive aufgenommen werden, eine verbesserte räumliche Wahrnehmung des Objektfelds zu ermöglichen. Die beiden Bilder, die gemeinsam das stereoskopische Bild darstellen, werden auch als "Halbbilder" oder als "Stereo-Halbbilder" bezeichnet. Die beiden Halbbilder werden separat dem rechten und dem linken Auge des Operateurs dargestellt, so dass dieser einen räumlichen Eindruck des Objektfelds gewinnen kann. Hierfür kann beispielsweise ein für eine stereoskopische Darstellung geeigneter Monitor vorgesehen sein, etwa ein Bildschirm mit einer wechselnden Polarisierung, wobei der Operateur eine Polarisationsbrille mit unterschiedlichen Polarisierungen der beiden Gläser trägt.

Ferner ist es bekannt, zur Verbesserung der Bildqualität oder für unterschiedliche Beobachtungsmodi unterschiedliche Filter in einen oder mehrere Strahlengänge eines optischen Beobachtungsinstruments einbringen zu können. Insbesondere ist es bekannt, dass zur Fluoreszenzdetektion eine Fluoreszenzanregungsstrahlung auf ein zu untersuchendes Gewebe eingestrahlt wird und die daraufhin von dem Gewebe emittierte Fluoreszenzstrahlung, die in der Regel bei größeren Wellenlängen als die eingestrahlte Fluoreszenzanregungsstrahlung liegt, beobachtet wird. Dabei kann sowohl die Autofluoreszenz des Gewebes als auch die Fluoreszenzemission von gezielt vorher dem Körper zugeführten Fluoreszenzfarbstoffen bzw. Photosensibilisatoren beobachtet werden. Da vom Gewebe auch Fluoreszenzanregungsstrahlung zurückgestrahlt wird, welche typischerweise deutlich heller als die Fluoreszenzstrahlung ist, ist es notwendig, die rückgestrahlte Fluoreszenzanregungsstrahlung zu blockieren, wofür je nach Fluoreszenzmodus ein passender Filter in einen Beobachtungsstrahlengang eingebracht werden muss.

Die Beobachtung der Fluoreszenzstrahlung ermöglicht eine verbesserte Unterscheidung von Gewebearten bzw. eine verbesserte Erkennung von erkranktem Gewebe, wobei für unterschiedliche Gewebe jeweils entsprechend unterschiedliche Photosensibilisatoren vorteilhaft sind. Um beispielsweise differenziert zwischen vitalem und nekrotischem Tumorgewebe, Normalgewebe sowie Blutgefäßen unterscheiden und die Aktivität der jeweiligen Gewebearten und/oder Zellen bestimmen zu können, ist es insbesondere wünschenswert, drei verschiedene Fluoreszenzmodi beobachten zu können, wofür drei spektral unterschiedliche, wechselbare Filter notwendig sind.

In DE 20 2011 000 688 U1 ist eine Vorrichtung zur Aufnahme von Filtern für Mikroskope offenbart, die zwei unabhängig voneinander um dieselbe Drehachse drehbare Filterräder aufweist, wobei die beiden Filterräder in axialer Richtung der Drehachse gesehen relativ zueinander versetzt sind. Die Vorrichtung hat eine Antriebseinheit zum Drehen des ersten Filterrads und zum Drehen des zweiten Filterrads um die Drehachse. Beide Filterräder weisen jeweils eine Vielzahl von Aufnahmebereichen zur Aufnahme jeweils mindestens eines Filters und einen Durchlassbereich zum ungefilterten Durchlassen von Licht auf. Die Vorrichtung kann ein drittes Filterrad umfassen, das ebenfalls um die Drehachse unabhängig von den anderen beiden Filterrädern drehbar ist. Diese Vorrichtung ist nicht für ein stereoskopisches Beobachtungsinstrument geeignet.

Aus EP 3 197 146 A1 ist ein medizinisches Beobachtungsgerät bekannt, bei dem mehrere Filter auf der äußeren Umfangsfläche eines schwenkbaren Halterahmens angeordnet sind, der um eine Rotationsachse rotierbar ist, welche senkrecht zur Richtung einer optischen Achse steht. Nachteilig hierbei ist, dass der Halterahmen beim Rotieren außerhalb der vertikalen Ebene viel Raum benötigt.

Gemäß EP 3 073 307 A1 umfasst eine Vorrichtung zum Aufnehmen eines Bildes eines Objektfelds an einem menschlichen oder tierischen Körper von außerhalb des Körpers eine Beobachtungsoptik zum Aufnehmen des Bildes des Objektfelds, die einen ersten und einen zweiten Stereokanal umfasst und um eine zu einer Blickrichtung der Beobachtungsoptik zumindest näherungsweise parallele Drehachse drehbar ist. Die Beobachtungsoptik umfasst ein erstes Filter, das in einen Strahlengang des ersten Stereokanals einschwenkbar und aus diesem ausschwenkbar ist, und ein zweites Filter, das in einen Strahlengang des zweiten Stereokanals einschwenkbar und aus diesem ausschwenkbar ist. Das erste und das zweite Filter sind auf einem gemeinsamen, um eine senkrecht zur Drehachse schwenkbaren Filterträger angeordnet, oder das erste und das zweite Filter sind jeweils in einem Filterträger aufgenommen, wobei eine Filterwelle des Filterträgers des zweiten Stereokanals über ein Getriebe von der Filterwelle des Filterträgers des ersten Stereokanals angetrieben wird.

EP 1 538 471 A1 offenbart eine Wechselvorrichtung für optische Elemente in Stereomikroskopen, mit der ein Paar optischer Elemente in die beiden Stereokanäle eines Stereomikroskops einbringbar ist. Dieses Dokument offenbart keine drei Filterräder, die entlang einer gemeinsamen Achse hintereinander angeordnet und um die gemeinsame Achse und zueinander rotierbar sind.

Es ist Aufgabe der vorliegenden Erfindung, den Stand der Technik zu verbessern. Insbesondere ist es Aufgabe der Erfindung, eine Filterwechselvorrichtung für ein optisches Be-obachtungsinstrument mit zwei Strahlengängen anzugeben, insbesondere für ein stereoskopisches Beobachtungsinstrument wie etwa ein Stereo-Videoendoskop, ein Stereo-Exoskop oder ein Stereo-Operationsmikroskop, welche Filterwechselvorrichtung das Wechseln von drei unterschiedlichen Filtern ermöglicht und einen geringeren Raumbedarf hat. Ferner ist es Aufgabe der Erfindung, ein entsprechendes optisches Beobachtungsinstrument mit zwei Strahlengängen sowie ein verbessertes Verfahren zum Wechseln eines Filters eines optischen Beobachtungsinstruments mit zwei Strahlengängen anzugeben.

Diese Aufgabe wird durch eine Filterwechselvorrichtung gemäß Anspruch 1, durch ein optisches Beobachtungsinstrument gemäß Anspruch 16 sowie durch ein Verfahren gemäß Anspruch 17 gelöst.

Vorteilhafte Weiterbildungen der Erfindung ergeben sich aus den Unteransprüchen.

Eine erfindungsgemäße Filterwechselvorrichtung für ein optisches Beobachtungsinstrument mit zwei Strahlengängen ist insbesondere als Filterwechselvorrichtung für ein stereoskopisches Beobachtungsinstrument, beispielsweise für ein Stereo-Videoendoskop, ein Stereo-Exoskop oder ein Stereo-Operationsmikroskop, ausgebildet. Das stereoskopische Beobachtungsinstrument kann beispielsweise zwei Bildsensoren für eine stereoskopische Fluoreszenzbeobachtung und zwei Bildsensoren für eine stereoskopische Weißlichtbeobachtung aufweisen. Die Filterwechselvorrichtung ist insbesondere eine Vorrichtung, welche einen Wechsel eines oder mehrerer sich jeweils im Strahlengang befindlicher Filter ermöglicht, insbesondere einen Wechsel von Beobachtungsfiltern. Hierbei ist die Filterwechselvorrichtung vorzugsweise ausgebildet, um wechselweise Filter mit drei unterschiedlichen spektralen Filtercharakteristiken in die zwei Strahlengänge einzubringen und daraus wieder zu entfernen. Die beiden Strahlengänge sind dabei insbesondere die Strahlengänge der beiden Stereokanäle des stereoskopischen Beobachtungsinstruments bzw. des Stereo-Videoendoskops, des Stereo-Exoskops oder des Stereo-Operationsmikroskops. Die Strahlengänge verlaufen vorzugsweise parallel zueinander, insbesondere in Bereich der Filterwechselvorrichtung, können aber auch miteinander einen Winkel bilden. Die Strahlengänge können in Bereich der Filterwechselvorrichtung beispielsweise afokal sein.

Erfindungsgemäß weist die Filterwechselvorrichtung ein erstes Filterrad, ein zweites Filterrad und ein drittes Filterrad auf, wobei die Filterräder entlang einer gemeinsamen Achse hintereinander angeordnet sind, insbesondere in axialer Richtung versetzt und beispielsweise miteinander fluchtend. Die Bezeichnungen "erstes", "zweites" und "drittes" Filterrad dienen dabei lediglich der Unterscheidung der Filterräder und sind hinsichtlich der Anordnung der Filterräder nicht einschränkend zu verstehen; insbesondere wird hierdurch nicht notwendig eine Reihenfolge der Filterräder in den Strahlengängen bezeichnet. So kann beispielsweise das erste, das zweite oder das dritte Filterrad in den Strahlengängen zuerst angeordnet sein, und die beiden anderen Filterräder können in unterschiedlicher Reihenfolge folgen. Das erste, das zweite und das dritte Filterrad sind um die gemeinsame Achse und jeweils zueinander rotierbar. Das erste, das zweite und das dritte Filterrad sind insbesondere um eine jeweilige Drehachse rotierbar, wobei die Drehachsen der Filterräder zumindest näherungsweise mit der gemeinsamen Achse fluchten.

Die gemeinsame Achse ist vorzugsweise in dem optischen Beobachtungsinstrument derart angeordnet oder anordenbar, dass sie im Wesentlichen parallel zu den beiden Strahlengängen verläuft und/oder eine Mittelachse zwischen den beiden Strahlengängen darstellt. Die gemeinsame Achse wird insbesondere durch ein Maschinenelement zur Lagerung der rotierbaren Filterräder gebildet, das nicht selbst rotieren muss und kein Drehmoment überträgt. Eine solche feststehende Achse kann somit auch eine gemeinsame Drehachse der Filterräder bilden. Über die gemeinsame Achse kann insbesondere auch eine fluchtende Anordnung der Filterräder hintereinander entlang der gemeinsamen Achse sichergestellt werden. Dazu weist beispielsweise jedes Filterrad eine mittige Bohrung auf, wobei die gemeinsame Achse durch diese mittigen Bohrungen der Filterräder geführt ist und somit die Filterräder mit ihren jeweiligen Drehachsen fluchtend in einer geraden Linie liegen. Die Filterräder können beispielsweise jeweils mittels eines Gleit-, Wälz- oder Kugellagers drehbar gelagert sein. Die Filterräder können unterschiedliche Durchmesser und/oder Formen am äußeren Umfang jedes Filterrades aufweisen. Eines oder mehrere Filterräder können beispielsweise mit näherungsweise kreisförmigem oder nicht-kreisförmigem Umfang oder auch als unterbrochene Filterhalter ausgebildet sein.

Das erste, das zweite und das dritte Filterrad weisen jeweils mindestens einen Filter und mindestens einen freien optischen Durchgang auf, wobei in jeden der beiden Strahlengänge ein Filter oder ein freier optischer Durchgang eines jeden Filterrads einbringbar ist. Insbesondere sind die Filter und die freien optischen Durchgänge in den Filterrädern derart angeordnet und die Filterräder derart zueinander rotierbar, dass in den ersten der beiden Strahlengänge ein Filter oder ein freier optischer Durchgang des ersten Filterrads und zugleich ein Filter oder ein freier optischer Durchgang des zweiten Filterrads und weiterhin ein Filter oder ein freier optischer Durchgang des dritten Filterrads einbringbar ist, wobei nicht alle Kombinationen der Filter und freien optischen Durchgänge der drei Filterräder notwendig realisierbar sein müssen. Entsprechendes gilt für den zweiten der beiden Strahlengänge. Vorzugsweise sind die Filter und die freien optischen Durchgänge derart angeordnet und die Filterräder derart rotierbar, dass jeweils zwei gleichartige Filter einander gegenüberliegend in die beiden Strahlengänge einbringbar und daraus wieder entfernbar sind.

Ein solcher Filter ist insbesondere dazu ausgebildet, einfallende Strahlung nach der Wellenlänge im optischen Wellenlängenbereich oder in angrenzenden Wellenlängenbereichen zu selektieren und einen Anteil hindurchtreten zu lassen, während ein anderer Anteil blockiert wird. Unter "gleichartigen Filtern" wird insbesondere verstanden, dass die gleichartigen Filter eine gleiche Selektion der einfallenden Strahlung vornehmen, beispielsweise gleiche spektrale Filtercharakteristiken haben. Unter einem "freien optischen Durchgang" wird hier ein optisches Element und/oder ein Bereich des jeweiligen Filterrads verstanden, welches bzw. welcher Strahlung frei von einer Selektion passieren lässt, insbesondere frei von einer spektralen Selektion. Ein freier optischer Durchgang kann daher beispielsweise eine Durchgangsöffnung oder auch ein spektral nicht selektives optisches Element sein.

Die Filterräder können beispielsweise jeweils als Scheibe ausgebildet sein, wobei ein oder mehrere Filter sowie ein oder mehrere freie Durchgänge gleichmäßig zueinander beabstandet und mit gleichem Abstand von der jeweiligen bzw. der gemeinsamen Drehachse angeordnet sein können. Ein Filterrad kann beispielsweise zwei Filter und zwei freie Durchgänge aufweisen, welche jeweils gegenüberliegend und zueinander um 90° versetzt angeordnet sind. Die Filter und die freien Durchgänge sind insbesondere in das Filterrad einschraubbar oder einsteckbar und haben vorzugsweise eine Form und eine Größe, die näherungsweise einem jeweiligen Querschnitt der beiden Strahlengänge entspricht, und sind beispielsweise jeweils kreisförmig ausgebildet. Die Filter sowie die freien optischen Durchgänge haben insbesondere auf dem Filterrad eine vorgegebene Filterposition.

Erfindungsgemäß ist eines der Filterräder antreibbar, insbesondere ist nur eines der Filterräder antreibbar, beispielsweise mittels eines Antriebs. Das antreibbare Filterrad wird in der vorliegenden Anmeldung als das "zweite" Filterrad bezeichnet; dieses kann in den Strahlengängen vor, zwischen oder nach dem ersten und dem dritten Filterrad angeordnet sein. Somit ist insbesondere nur das zweite Filterrad mittels des Antriebs antreibbar, welcher Antrieb nicht an dem ersten und dem dritten Filterrad angreift. Weiter erfindungsgemäß ist das erste Filterrad über einen ersten Mitnehmer und das dritte Filterrad über einen zweiten Mitnehmer mit dem zweiten Filterrad gekoppelt. Der Mitnehmer ist insbesondere ein Bauteil, welches bei seiner Bewegung ein anderes Filterrad ebenfalls in Drehbewegung versetzt und somit mitnimmt oder, je nach Rotationsrichtung und Stellung der Filterräder, stehen lässt. Insbesondere ist das erste Filterrad derart über den Mitnehmer mit dem zweiten Filterrad gekoppelt, dass beim Rotieren des zweiten Filterrades das erste Filterrad mittels des Mitnehmer mitnehmbar ist und somit ebenfalls in der Rotationsrichtung des zweiten Filterrads rotiert, oder auch freilaufbar ist und somit stehen gelassen wird; entsprechend ist das dritte Filterrad insbesondere derart über den Mitnehmer mit dem zweiten Filterrad gekoppelt, dass beim Rotieren des zweiten Filterrades in einer Rotationsrichtung das dritte Filterrad mittels des Mitnehmer mitnehmbar ist oder freilaufbar ist.

Somit wird eine Filterwechselvorrichtung bereitgestellt, bei welcher durch optimale Anordnung und Bewegungsverbindung von drei Filterrädern mindestens drei verschiedene Filterarten, insbesondere Filter mit drei unterschiedlichen spektralen Filtercharakteristiken, in die Strahlengänge unter Beanspruchung eines geringen Bauraums einwechselbar sind. Es ist besonders vorteilhaft, dass die Filterwechselvorrichtung hierbei im Wesentlichen nur einen Bauraum einnimmt, welcher auch von einem einzelnen Filterrad beansprucht würde. Insbesondere kann dadurch, dass drei Filterräder in Strahlrichtung fluchtend hintereinander angeordnet sind, ein Wechsel von drei unterschiedlichen Filtern in den beiden Strahlengängen realisierbar sein, wobei die Filterräder jeweils einen Außendurchmesser aufweisen, der gleich oder nur geringfügig größer als der doppelte Durchmesser jedes Filters ist, und somit auch die Filterwechselvorrichtung besonders kompakt ausgestaltet sein kann.

Zudem ist in der Filterwechselvorrichtung ein schneller Wechsel der Filter realisierbar, da die Filterräder bereits quer zur Richtung der Strahlengänge liegen und nur gedreht werden müssen, um das jeweils gewünschte Filter in den Strahlengang einzubringen. Dadurch wird eine zeitgleiche oder quasi zeitgleiche bildliche Darstellung von unterschiedlichen aufgenommenen Fluoreszenzen und/oder Weißlichtbildern ermöglicht.

Weiterhin wird dadurch, dass drei Filterräder hintereinander koaxial angeordnet sind, die von den beiden Strahlengängen durchsetzt werden, wobei lediglich eines der Filterräder antreibbar ist, welches hier als das zweite Filterrad bezeichnet wird und das über seine Mitnehmer dem ersten Filterrad und/oder dem dritten Filterrad eine Rotation aufprägen kann, um entsprechende Filter einzuwechseln, ein besonders einfacher Aufbau ermöglicht. Insbesondere ist nur ein einziger Antrieb notwendig, und lediglich das zweite Filterrad muss dazu ausgestaltet sein, dass der Antrieb an diesem angreifen kann. Auch hierdurch kann eine besonders raumsparende Anordnung erreichbar sein.

Gemäß einer bevorzugten Ausführungsform der Erfindung ist das antreibbare Filterrad, d.h. das zweite Filterrad, zwischen dem ersten und dem dritten Filterrad angeordnet, beispielsweise entlang der gemeinsamen Achse gesehen etwa mittig zwischen dem ersten und dem dritten Filterrad. Dadurch, dass nur das mittlere Filterrad antreibbar ist und das davor und das danach angeordnete Filterrad über den ersten bzw. den zweiten Mitnehmer mit diesem gekoppelt sind, ist eine besonders einfache Anordnung und Ausgestaltung der Filterräder erreichbar, die einen besonders sicheren und präzisen Filterwechsel ermöglicht.

Vorzugsweise ist das erste Filterrad über den ersten Mitnehmer derart mit dem zweiten Filterrad gekoppelt, dass ausgehend von einer Ausgangsposition das erste Filterrad bei einer Rotation des zweiten Filterrads in einer ersten Rotationsrichtung des zweiten Filterrads mitgenommen und bei einer Rotation des zweiten Filterrads in einer zweiten, der ersten entgegengesetzten Rotationsrichtung stehengelassen wird. In Abhängigkeit von einer Rotationsrichtung des zweiten Filterrads und einer Ausgangs-Drehposition des ersten und des zweiten Filterrads, insbesondere einer relativen Ausgangsposition des ersten bezogen auf das zweite Filterrad, wird somit das erste Filterrad von dem zweiten mitgenommen oder nicht.

In bevorzugter Weise ist das dritte Filterrad über den zweiten Mitnehmer derart mit dem zweiten Filterrad gekoppelt, dass ausgehend von einer Ausgangsposition das dritte Filterrad bei einer Rotation des zweiten Filterrads in einer ersten Rotationsrichtung des zweiten Filterrads stehengelassen und bei einer Rotation des zweiten Filterrads in einer zweiten, der ersten entgegengesetzten Rotationsrichtung mitgenommen wird. In Abhängigkeit von einer Rotationsrichtung des zweiten Filterrads und einer Ausgangs-Drehposition des zweiten und des dritten Filterrads, insbesondere einer relativen Ausgangsposition des dritten bezogen auf das zweite Filterrad, wird somit das erste Filterrad von dem zweiten mitgenommen oder nicht.

In besonders bevorzugter Weise ist das erste Filterrad über den ersten Mitnehmer derart mit dem zweiten Filterrad gekoppelt, dass ausgehend von einer Ausgangsposition das erste Filterrad bei einer Rotation des zweiten Filterrads in einer ersten Rotationsrichtung des zweiten Filterrads mitgenommen und in einer zweiten, der ersten entgegengesetzten Rotationsrichtung stehengelassen wird, und zugleich das dritte Filterrad über den zweiten Mitnehmer derart mit dem zweiten Filterrad gekoppelt ist, dass ausgehend von einer Ausgangsposition das dritte Filterrad bei einer Rotation des zweiten Filterrads in der zweiten Rotationsrichtung mitgenommen und in der ersten Rotationsrichtung stehengelassen wird.

Hierdurch kann auf besonders einfache Weise eine Verstellung des ersten, zweiten und/oder dritten Filterrads zueinander und somit ein Einschwenken der entsprechenden Filter in die beiden Strahlengänge durch Rotation des zweiten Filterrads in der ersten oder der zweiten Rotationsrichtung erreicht werden.

Gemäß einer bevorzugten Ausführungsform der Erfindung ist der erste Mitnehmer als Mitnehmerstift ausgebildet, welcher an dem zweiten Filterrad angeordnet ist und in eine Aussparung des ersten Filterrades eingreift, und/oder der zweite Mitnehmer ist als Mitnehmerstift ausgebildet, welcher an dem zweiten Filterrad angeordnet ist und in eine Aussparung des dritten Filterrades eingreift, wobei der erste bzw. der zweite Mitnehmer in der jeweiligen Aussparung in einem jeweiligen Freilaufwinkelbereich freilaufbar ist. Der Mitnehmerstift ist insbesondere ein stiftförmiges, zylinder- oder kegelförmiges Bauteil, welches in eine jeweilige Aussparung eingreift und in dieser beweglich geführt ist. Somit kann durch Eingreifen des jeweiligen Mitnehmerstiftes in eine Aussparung des ersten Filterrades und/oder des dritten Filterrades direkt ein Mitnehmen und somit eine Rotationsbewegung des ersten Filterrades und/oder des dritten Filterrades realisiert werden. Vorzugsweise sind die Freilaufwinkelbereiche des ersten und des zweiten Mitnehmers näherungsweise gleich und betragen beispielsweise etwa 90°. Die Länge der Aussparung, in welcher der Mitnehmer beweglich ist, kann somit beispielsweise einem Winkelbereich von 90° entsprechen. In dem Fall, dass sich das erste und das dritte Filterrad auf derselben Seite des zweiten Filterrads befinden und beispielsweise das erste zwischen dem zweiten und dem dritten Filterrad angeordnet ist, kann der zweite Mitnehmer zum Mitnehmen des dritten Filterrads durch eine entsprechend ausgestaltete Aussparung des ersten Filterrads hindurchgreifen oder auch beispielsweise über einen äußeren Umfang des ersten Filterrads um dieses herumgreifen.

Hierdurch kann auf besonders einfache Weise erreicht werden, dass das erste Filterrad ausgehend von einer Ausgangsposition bei einer Rotation des zweiten Filterrads in der ersten Rotationsrichtung mitgenommen wird und bei einer Rotation des zweiten Filterrads in der zweiten, entgegengesetzten Rotationsrichtung bis zu einem Freilaufwinkel von beispielsweise 90° stehen gelassen wird. Dementsprechend kann hierdurch erreicht werden, dass das dritte Filterrad ausgehend von einer Ausgangsposition bei einer Rotation des zweiten Filterrads in der zweiten Rotationsrichtung mitgenommen wird und bei einer Rotation des zweiten Filterrads in der ersten Rotationsrichtung bis zu dem Freilaufwinkel stehen gelassen wird. Folglich können allein durch Antreiben des zweiten Filterrades das erste Filterrad und/oder das dritte Filterrad mitgenommen oder stehen gelassen werden, um gewünschte Filterschwenkpositionen einzustellen, ohne dass separate Antriebe für jedes Filterrad notwendig sind. Dadurch ist ein sehr geringer Bauraum der Filterwechselvorrichtung realisierbar.

Der erste bzw. der zweite Mitnehmer können fest an dem zweiten Filterrad angeordnet sein. Der erste und der zweite Mitnehmer können einstückig, insbesondere als ein die Scheibe des zweiten Filterrads durchgreifender Stift, ausgebildet sein und am einen Ende in die Aussparung des ersten Filterrades und am anderen Ende in die Aussparung des dritten Filterrades eingreifen. Vorzugsweise weist das zweite Filterrad zwei separate Mitnehmerstifte auf, welche auf einer jeweiligen Seite des zweiten Filterrads vorstehen und jeweils in die Aussparungen des ersten Filterrades und des dritten Filterrades eingreifen.

In entsprechender Weise kann es vorgesehen sein, dass der erste Mitnehmer an dem ersten Filterrad angeordnet ist und in eine Aussparung des zweiten Filterrads eingreift und/oder der zweite Mitnehmer an dem dritten Filterrad angeordnet ist und in eine Aussparung des zweiten Filterrads eingreift, wodurch ebenfalls erreicht werden kann, dass das erste und/oder das dritte Filterrad in der beschriebenen Weise vom zweiten Filterrad mitgenommen bzw. stehen gelassen werden.

Die Aussparung des ersten und/oder des dritten Filterrads ist insbesondere jeweils als ringsegmentförmige Nut oder als ringsegmentförmiger Schlitz in einer Scheibe, die das jeweilige Filterrad bildet, ausgebildet. Sofern im zweiten Filterrad eine Aussparung vorgesehen ist, kann diese ebenfalls als ringsegmentförmige Nut bzw. ringsegmentförmiger Schlitz ausgebildet sein, und ebenso in dem Fall, dass im zweiten Filterrad zwei Aussparungen vorgesehen sind, können diese jeweils durch ringsegmentförmige Nuten oder Schlitze gebildet werden. Die jeweilige Nut bzw. der jeweilige Schlitz erstreckt sich insbesondere, von der gemeinsamen Achse aus gesehen, in einer Scheibe des jeweiligen Filterrads bei einem konstanten Radius und um einen Winkel, der näherungsweise dem Freilaufwinkel entspricht bzw. um so viel größer ist, dass der Mitnehmer sich in der Nut bzw. in dem Schlitz um den Freilaufwinkel frei bewegen kann. Bevorzugt hat die Nut bzw. der Schlitz eine Länge entsprechend eines Umfanges eines Viertelkreises um die gemeinsame Drehachse und erstreckt sich somit über einen Winkelbereich von näherungsweise 90°.

Die Aussparung ist auf dem jeweiligen Filterrad insbesondere so angeordnet, dass diese nicht die optischen Eigenschaften der Filter beeinträchtigt. Vorzugsweise erstreckt sich die Nut bzw. der Schlitz in der Scheibe eines jeweiligen Filterrads durch einen freien Durchgang des Filterrads hindurch. Auf diese Weise kann ein besonders einfacher und kompakter Aufbau zum Rotieren des ersten und des dritten Filterrads erreichbar sein, ohne die optische Funktion der Filter zu beeinträchtigen.

Um nach Rotieren des ersten und/oder des dritten Filterrads durch Mitnehmen mittels des ersten bzw. des zweiten Mitnehmers ein Zurückkehren des ersten und/oder des zweiten Filterrads zu ermöglichen und insbesondere das erste und/oder das dritte Filterrad in seine jeweilige Ausgangsdrehposition zurückzusetzen, weist oder weisen das erste Filterrad und/oder das dritte Filterrad eine Rückstelleinrichtung auf. Die Rückstelleinrichtung ist dabei insbesondere ein Maschinenelement, das zum Ausüben eines Drehmoments auf das erste Filterrad bzw. das dritte Filterrad in Richtung auf seine jeweilige Ausgangsposition ausgebildet ist, so dass das erste bzw. das dritte Filterrad, wenn es frei von einer Mitnahme ist, in die jeweilige Ausgangsposition zurückversetzt wird. Die Rückstelleinrichtung kann beispielsweise eine Rückstellfeder sein, welche sich federgespannt sowohl auf einer feststehenden Achse oder an einem feststehenden Halter, als auch an einer Oberfläche des ersten Filterrades und/oder des dritten Filterrades abstützt. Hierdurch kann auf besonders einfache Weise eine Rotation des ersten und/oder des dritten Filterrads in einem Freilaufwinkelbereich unabhängig von einer Mitnahme und somit einer Rotation des zweiten Filterrads, und somit eine Einstellung entsprechender Filterschwenkpositionen, ermöglicht werden.

Gemäß einer bevorzugten Ausführungsform der Erfindung weisen das erste, das zweite und/oder das dritte Filterrad jeweils zwei Filter mit einer ersten, zweiten bzw. dritten Filtercharakteristik und jeweils zwei freie optische Durchgänge auf. Somit weist das erste Filterrad zwei Filter mit einer ersten Filtercharakteristik und zwei freie optische Durchgänge auf, und/oder das zweite Filterrad weist zwei Filter mit einer zweiten Filtercharakteristik und zwei freie optische Durchgänge auf, und/oder das dritte Filterrad weist zwei Filter mit einer dritten Filtercharakteristik und zwei freie optische Durchgänge auf. In besonders bevorzugter Weise umfassen das erste, das zweite und das dritte Filterrad jeweils zwei Filter mit einer ersten, zweiten bzw. dritten Filtercharakteristik und jeweils zwei freie optische Durchgänge. Somit weist vorzugsweise jedes Filterrad zwei jeweils gleichartige Filter und zwei freie optische Durchgänge auf. Dadurch, dass ein Filterrad oder jedes Filterrad jeweils genau zwei gleichartige Filter aufweist, ist ein schneller und präziser Wechsel der Filter realisierbar, wobei in beiden Strahlengängen ein gleichartiger Filter eingebracht werden kann.

Gemäß einer besonders bevorzugten Ausführungsform der Erfindung weisen das erste, das zweite und/oder das dritte Filterrad jeweils zwei einander gegenüberliegend angeordnete Filter mit einer ersten, zweiten bzw. dritten Filtercharakteristik und jeweils zwei, ebenfalls einander gegenüberliegend angeordnete, freie optische Durchgänge auf. Somit weist das erste Filterrad zwei einander gegenüberliegende Filter mit einer ersten Filtercharakteristik und zwei einander gegenüberliegende freie optische Durchgänge auf, und/oder das zweite Filterrad weist zwei einander gegenüberliegende Filter mit einer zweiten Filtercharakteristik und zwei einander gegenüberliegende freie optische Durchgänge auf, und/oder das dritte Filterrad weist zwei einander gegenüberliegende Filter mit einer dritten Filtercharakteristik und zwei einander gegenüberliegende freie optische Durchgänge auf. In besonders bevorzugter Weise umfassen das erste, das zweite und das dritte Filterrad jeweils zwei einander gegenüberliegend angeordnete Filter mit einer ersten, zweiten bzw. dritten Filtercharakteristik und jeweils zwei einander gegenüberliegend angeordnete, freie optische Durchgänge. Somit weist vorzugsweise jedes Filterrad zwei jeweils gleichartige gegenüberliegende Filter und zwei gegenüberliegende freie optische Durchgänge auf. Das Gegenüberliegen der Filter bzw. freien optischen Durchgänge bezieht sich dabei jeweils auf die Drehachse des betreffenden Filterrads bzw. auf die gemeinsame Achse. Dadurch, dass ein Filterrad oder jedes Filterrad jeweils genau zwei gleichartige gegenüberliegende Filter aufweist, ist ein besonders schneller und präziser Wechsel der Filter realisierbar, wobei in beiden Strahlengängen ein gleichartiger Filter eingebracht werden kann.

Alternativ kann es vorgesehen sein, dass bei einem oder mehreren Filterrädern jeweils zwei Filter, insbesondere zwei gleichartige Filter, nebeneinander und zwei Durchgänge nebeneinander angeordnet sind, wobei die beiden Filter gleichzeitig in die beiden Strahlengänge oder die beiden Durchgänge gleichzeitig in die beiden Strahlengänge einbringbar sind. Wenn beispielsweise in einer oberen Hälfte eines Filterrads beide Filter angeordnet sind, können diese in einer ersten Drehposition jeweils in einem Strahlengang liegen und in einer zweiten, um 180° gedrehten Drehposition des Filterrads außerhalb der Strahlengänge, wobei in der zweiten Drehposition die beiden Strahlengänge jeweils durch einen freien Durchgang verlaufen. Für einen Filterwechsel würde in diesem Fall eine Rotation bzw. eine Mitnahme eines Filterrads um 180° erfolgen bzw. ein Freilaufwinkel 180° betragen.

In einer weiteren Ausführungsform kann es vorgesehen sein, dass mindestens zwei unterschiedliche Filterräder jeweils einen gleichartigen Filter aufweisen, wobei die beiden gleichartigen Filter und jeweils ein freier optischer Durchgang der beiden Filterräder derart zueinander anordenbar sind, dass die beiden gleichartigen Filter sowie die jeweiligen freien optischen Durchgänge einander gegenüberliegend in die beiden Strahlengänge einbringbar sind. Auch hierdurch kann es erreicht werden, dass die beiden Strahlengänge durch gleichartige Filter hindurchtreten.

Insbesondere sind die erste, die zweite und die dritte Filtercharakteristik zur spektralen Selektion der auftreffenden Strahlung ausgebildet, wobei die erste, die zweite und die dritte Filtercharakteristik vorzugsweise unterschiedliche spektrale Filtercharakteristiken sind.

Um das Beobachten und/oder die Bildaufnahme in verschiedenen Fluoreszenzmodi zu ermöglichen, ist oder sind einer oder mehrere der Filter als Fluoreszenzfilter ausgebildet. Insbesondere sind die Filter mit der ersten, zweiten und/oder dritten Filtercharakteristik als Fluoreszenzfilter ausgebildet, besonders bevorzugt sind die Filter mit der ersten, zweiten und dritten Filtercharakteristik jeweils Fluoreszenzfilter. Ein Fluoreszenzfilter weist insbesondere eine derartige Filtercharakteristik auf, dass für einen jeweiligen Fluoreszenzmodus Strahlung im Wellenlängenbereich der jeweils spezifischen Fluoreszenzanregungsstrahlung zumindest weitgehend blockiert wird und im Wellenlängenbereich der jeweils spezifischen Fluoreszenzemissionsstrahlung zumindest weitgehend durchgelassen wird. Bei einem solchen Filter kann es sich etwa um einen Bandpassfilter, einen Bandsperrfilter, einen Kantenfilter oder einen Interferenzfilter handeln, beispielsweise um einen optischen polychroitischen Interferenzfilter, wodurch eine Selektion der emittierten Fluoreszenzstrahlung gegenüber der Anregungsstrahlung möglich ist.

In besonders bevorzugter Weise sind die Filter mit der ersten, zweiten und dritten Filtercharakteristik als Fluoreszenzfilter für jeweils unterschiedliche Fluoreszenzmodi ausgebildet. Hierdurch kann es erreicht werden, dass die Filterwechselvorrichtung die Beobachtung von drei unterschiedlichen Fluoreszenzmodi und somit eine besonders weitgehende Unterscheidung unterschiedlicher Gewebearten bzw. von normalem, krankem und nekrotischem Gewebe ermöglicht.

Beispielsweise kann zur Beobachtung und Aufnahme der Fluoreszenz von Indocyaningrün (ICG) eines der Fluoreszenzfilter eine hohe Transmission in einem Wellenlängenbereich von circa 800 nm bis 950 nm haben, in welchem das Fluoreszenzlicht der ICG-Fluoreszenz emittiert wird, und in einem Wellenlängenbereich um 785 nm weitgehend sperren, in welchem Bereich üblicherweise eine Anregungsstrahlung der ICG-Fluoreszenz liegt. Weiter kann das Filter in einem Wellenlängenbereich von etwa 400 nm bis 660 nm eine hohe Transmission haben. Hierdurch kann es erreichbar sein, dass mit dem Fluoreszenzfilter nicht nur die ICG-Fluoreszenz, sondern auch im Weißlicht beobachtet werden kann. Zur Trennung des Weißlichts von der ICG-Fluoreszenzstrahlung kann nachfolgend in den beiden Strahlengängen jeweils ein dichroitischer Strahlteiler vorgesehen sein, wodurch die Strahlung im Bereich von 400 nm bis 660 nm auf einen ersten, dem jeweiligen Stereokanal zugeordneten Bildaufnehmer und die Fluoreszenzstrahlung auf einen zweiten, dem betreffenden Stereokanal zugeordneten Bildaufnehmer geführt werden kann.

Ein weiteres Fluoreszenzfilter kann eine Durchlässigkeit für Fluoreszenzlicht in einem Wellenlängenbereich von circa 500 nm bis 600 nm haben und in einem Wellenlängenbereich der entsprechenden Fluoreszenzanregungsstrahlung, insbesondere bei 405 nm, als Sperrfilter wirken; hierdurch kann die emittierte Fluoreszenz von Protoporphyrin IX (PPIX) aus angereichertem 5-ALA Farbstoff beobachtet werden, die auch als PDD (photodynamische Diagnose)-Fluoreszenz bezeichnet wird. Hierbei kann gleichzeitig die Autofluoreszenz von menschlichem oder tierischem Gewebe angeregt werden, wobei das Filter zur Beobachtung der Autofluoreszenzstrahlung zusätzlich in einem Wellenlängenbereich von ca. 410 bis 600 nm durchlässig sein kann. Dabei kann ebenfalls ein nachgeschalteter dichroitischer Strahlteiler zur separaten Beobachtung der PPIX- und der Autofluoreszenzstrahlung vorgesehen sein.

Um die Fluoreszenz von Fluorescein zu beobachten, was beispielsweise für die Erkennung von Blutgefäßen in einem Gewebe vorteilhaft ist, kann ein weiterer der Fluoreszenzfilter eine hohe Durchlässigkeit in einem Wellenlängenbereich von circa 500 nm bis 600 nm aufweisen und im Übrigen eine niedrige Transmission haben bzw. als Sperrfilter wirken.

Gemäß einer bevorzugten Ausführungsform der Erfindung ist das zweite Filterrad zur Rotation motorisch antreibbar. Dadurch, dass der Antrieb des zweiten Filterrades ein motorischer Antrieb ist, kann die Bedienung vereinfacht werden und/oder ein automatischer Filterwechsel realisiert werden, der beispielsweise durch eine Steuerungseinrichtung gesteuert werden kann. Insbesondere können hierdurch schnell und präzise vorgegebene Filterschwenkpositionen angefahren werden.

Vorzugsweise ist das zweite Filterrad der Filterwechselvorrichtung im Wesentlichen an seinem äußeren Umfang antreibbar. Dabei kann der Antrieb auf eine äußere Umfangsfläche bzw. einen auf dieser angeordneten Zahnkranz wirken oder auch auf eine Stirnfläche bzw. einen umlaufenden Rand der Scheibe des Filterrads einwirken. Dadurch, dass das zweite Filterrad über eine außen gelegene Oberfläche angetrieben wird, kann die Genauigkeit der Rotationsbewegung verbessert werden. Zudem kann es vermieden werden, dass ein Antrieb in oder zwischen den beiden Strahlengängen angeordnet werden muss, wodurch der Raumbedarf der Filterwechselvorrichtung erhöht oder die optische Qualität der Abbildungen verringert würde. Insbesondere kann auch eine mittige, sich drehende und angetriebene Rotationsachse vermieden werden, wodurch beispielsweise ein Spiel und/oder ein Verklemmen eines der Filterräder die Rotation der anderen Filterräder beeinflussen könnte.

In besonders bevorzugter Weise ist das zweite Filterrad an seinem äußeren Umfang mittels eines motorischen Antriebes antreibbar, insbesondere über ein motorisch angetriebenes Zahnrad, das in einen Zahnkranz eingreift, der sich über zumindest einen Teil des äußeren Umfangs des Filterrads erstreckt, beispielsweise über 180°. Hierdurch kann einerseits eine automatische, sichere und präzise Rotationsbewegung des zweiten Filterrads ermöglicht werden. Andererseits kann dadurch, dass der motorische Antrieb außen am zweiten Filterrad angeordnet ist, dieser bei einem Ausfall oder bei einem notwendigen Austausch leicht ersetzt werden, ohne dass die Filterräder von der gemeinsamen Achse demontiert werden müssen.

Gemäß einer bevorzugten Ausführungsform weist die Filterwechselvorrichtung eine Positionserkennungseinrichtung zum Bestimmen einer jeweiligen Drehposition mindestens eines der Filterräder, vorzugsweise des ersten und dritten Filterrades, besonders bevorzugt aller Filterräder, auf. Insbesondere ist die Positionserkennungseinrichtung zum Detektieren des Erreichens oder Nicht-Erreichens einer jeweils vorgegebenen Drehposition des jeweiligen Filterrades ausgebildet. Hierdurch kann feststellbar sein, ob ein Filterrad sich bei einem Filterwechsel noch in der Rotation befindet und noch nicht an einer jeweiligen Drehposition angekommen ist bzw. wann eine Rotation zum Ein- oder Ausschwenken eines oder mehrerer Filter abgeschlossen ist. Ferner kann es eindeutig identifizierbar sein, welcher Filter bzw. welche gleichartigen Filter aktuell in die Strahlengänge eingeschwenkt sind und somit beispielsweise welcher Fluoreszenzmodus aufgenommen wird. Weiterhin kann hierdurch ein Fehlerzustand detektierbar sein, etwa eine nicht ausgeführte oder nicht für einen Filterwechsel ausreichende Rotation eines oder mehrerer der Filterräder, beispielsweise ausgelöst durch ein Verklemmen beim Bewegen der Filterräder. Auf diese Weise kann die Sicherheit bei der Durchführung einer Operation mit dem optischen Beobachtungsinstrument erhöht werden. Hierfür kann die Filterwechselvorrichtung oder das optische Beobachtungsinstrument eine Steuerungseinrichtung aufweisen oder mit einer solchen verbindbar sein, die derart eingerichtet ist, dass aus Signalen der Positionserkennungseinrichtung auf die Ausführung einer Rotationsbewegung bzw. auf einen Fehlerzustand geschlossen werden kann.

Vorzugsweise weist eine Positionserkennungseinrichtung eine Lichtschranke oder mehrere Lichtschranken auf, und das Filterrad bzw. mehrere der Filterräder weisen jeweils eine zugehörige Fahne auf. Eine solche Lichtschranke ist insbesondere ein optoelektronisches System, welches die Unterbrechung eines Lichtstrahls erkennt und als elektrisches Signal anzeigt und/oder ausgibt. Dabei kann die jeweilige Fahne durch eine Rotation des betreffenden Filterrads in die Lichtschranke einfahren und dadurch einen Lichtstrahl der Lichtschranke unterbrechen, wodurch die betreffende Drehposition des Filterrads detektiert wird. Ergänzend oder alternativ zu einer Fahne können eines oder mehrere der Filterräder jeweils eine Aussparung an ihrem äußeren Umfang aufweisen, so dass jeweils einer der drei Filter von einer der Lichtschranken erfasst wird. Bevorzugt weist die Positionserkennungseinrichtung drei Lichtschranken auf, welche jeweils mindestens eine Fahne oder eine Aussparung eines der drei Filterräder detektieren. Somit können die Drehpositionen der Filterräder mittels einer oder mehrerer Lichtschranken berührungslos detektiert werden.

In bevorzugter Weise weist die Filterwechselvorrichtung eine mechanische Anschlagseinrichtung oder mehrere mechanische Anschlagseinrichtungen auf, bis zu der oder denen das erste, zweite und/oder dritte Filterrad rotierbar ist oder sind. Insbesondere können die eine oder mehreren Anschlagseinrichtungen ausgebildet sein, um eine End- und/oder Ausgangsposition einer Rotation eines jeweiligen Filterrads festzulegen bzw. einen Winkelbereich, innerhalb dessen das Filterrad rotieren kann, zu begrenzen. Hierdurch können die Genauigkeit und die Zuverlässigkeit des Filterwechsels verbessert werden.

In besonders bevorzugter Weise kann zusätzlich zu einer Anschlagseinrichtung eine Positionserkennungseinrichtung vorgesehen sein, die mindestens eine Lichtschranke umfasst, wobei mindestens eines der Filterräder eine zugehörige Fahne aufweist, wobei die Fahne zugleich als Anschlaghebel der Anschlagseinrichtung dient. Dabei können die Anschlagseinrichtungen derart ausgebildet sein, dass ein jeweiliger Anschlaghebel beim Einfahren in eine Lichtschranke oder nach Passieren der entsprechenden Lichtschranke auf einen jeweiligen Anschlagstift der mechanischen Anschlagseinrichtung auftrifft. Hierdurch kann ein besonders einfacher Aufbau ermöglicht werden.

Beispielsweise kann es vorgesehen sein, dass das zweite Filterrad eine halbkreisförmige Aussparung oder einen halbkreisförmig größeren Durchmesser aufweist, welcher größere Durchmesser auch etwa durch den auf dem äußeren Umfang angeordneten Zahnkranz realisiert sein kann, während das erste Filterrad und das dritte Filterrad versetzt jeweils einen Anschlaghebel als Fahne aufweisen. Die Positionen der drei Filterräder werden mit drei Lichtschranken überwacht, welche an dem äußeren Umfang der Filterräder angeordnet sind. Hierbei registriert eine erste Lichtschranke, wenn der Anschlaghebel des ersten Filterrades seinen Anschlag erreicht hat und eine dritte Lichtschranke, wenn der Anschlaghebel des dritten Filterrads seinen Anschlag erreicht hat. Eine zweite Lichtschranke registriert die Aussparung des zweiten Filterrades. So kann eine erste Filterposition beispielsweise dadurch detektiert werden, dass die erste Lichtschranke den Anschlaghebel des ersten Filterrades und die mittlere Lichtschranke die fehlende Aussparung des zweiten Filterrades registriert, eine zweite Filterposition dadurch, dass die erste und die zweite Lichtschranke jeweils die Anschlaghebel des ersten Filterrades und des dritten Filterrades sowie die mittlere Lichtschranke die Aussparung des zweiten Filterrades registrieren, und eine dritte Filterposition dadurch, dass die dritte Lichtschranke den Anschlaghebel des dritten Filterrades und die mittlere Lichtschranke die fehlende Aussparung des zweiten Filterrades registriert. Die Filterwechselvorrichtung oder das optische Beobachtungsinstrument kann eine Steuerungseinrichtung aufweisen oder mit einer solchen verbindbar sein, die derart eingerichtet ist, dass aus den Signalen der Lichtschranken auf die Positionen der Filterräder geschlossen werden kann. Dadurch kann eine sichere, redundante Positionserkennung der Filterräder gewährleistet werden.

In einem zusätzlichen Aspekt der Erfindung wird die Aufgabe gelöst durch ein optisches Beobachtungsinstrument, insbesondere ein stereoskopisches Beobachtungsinstrument, beispielsweise ein Stereo-Videoendoskop, ein Stereo-Exoskop oder ein Stereo-Operationsmikroskop, mit zwei Strahlengängen, wobei das optische Beobachtungsinstrument eine Filterwechselvorrichtung aufweist, die wie zuvor beschrieben ausgebildet ist. Im Übrigen kann das optische Beobachtungsinstrument ausgebildet sein wie in der am gleichen Tag wie die vorliegende Anmeldung eingereichten deutschen Patentanmeldung desselben Anmelders beschrieben ist, welche die Bezeichnung "Optisches Beobachtungsinstrument sowie Verfahren zum Erzeugen eines Stereobilds eines Objektfelds" hat.

Insbesondere ist die Filterwechselvorrichtung zum Einbringen oder Entfernen jeweils zweier gleichartiger Filter in die bzw. aus den beiden Strahlengängen ausgebildet. Das optische Beobachtungsinstrument kann zwei oder mehr Bildsensoren aufweisen, beispielsweise vier Bildsensoren für eine stereoskopische Fluoreszenzaufnahme und eine stereoskopische Weißlichtaufnahme. Besonders bevorzugt kann das optische Beobachtungsinstrument zum Beobachten von drei unterschiedlichen Fluoreszenzmodi sowie im Weißlicht ausgebildet sein. Ferner kann das optische Beobachtungsinstrument eine Beleuchtungsoptik zum Beleuchtung eines Objektfelds mit einer Beleuchtungsstrahlung, insbesondere einer Fluoreszenzanregungsstrahlung, aufweisen und hierfür eine entsprechende Lichtquelle umfassen oder an eine solche anschließbar sein. Weiter kann das optische Beobachtungsinstrument eine Steuerungseinrichtung aufweisen oder mit einer solchen verbindbar sein, die zur Steuerung einer motorischen Rotation des zweiten Filterrads sowie zur Erkennung einer jeweiligen Position der Filterräder bzw. eines Fehlerzustands mittels der Signale einer Positionserkennungseinrichtung eingerichtet ist.

Somit wird ein stereoskopisches Beobachtungsinstrument bereitgestellt, bei dem die Filterwechselvorrichtung in einem sehr geringen Bauraum einen schnellen und sicheren Wechsel von drei verschiedenen Filtern, insbesondere Fluoreszenzfiltern, und somit das Beobachten von drei Fluoreszenzmodi und zusätzlich Weißlicht ermöglicht. Folglich wird mittels des stereoskopischen Beobachtungsinstrumentes eine sehr differenzierte Betrachtung und Aufnahme von Gewebe ermöglicht.

Gemäß einem weiteren Aspekt der Erfindung umfasst ein Verfahren zum Wechseln eines Filters eines optischen Beobachtungsinstruments, das zwei Strahlengänge aufweist und welches eine Filterwechselvorrichtung aufweist, die wie zuvor beschrieben ausgebildet ist, insbesondere zum Wechseln jeweils gleichartiger Filter in beiden Strahlengängen, für einen ersten Filterwechsel die Schritte
- Antreiben des zweiten Filterrads aus einer Ausgangsposition des zweiten Filterrads zur Rotation um die gemeinsame Achse in einer ersten Rotationsrichtung um einen Verstellwinkel,
- Mitnehmen des ersten Filterrads aus einer Ausgangsposition des ersten Filterrads mittels des ersten Mitnehmers zur Rotation in der ersten Rotationsrichtung um den Verstellwinkel,
- Stehenlassen des dritten Filterrads in einer Ausgangsposition des dritten Filterrads.

Weiter können für einen weiteren Wechsel der Filter in beiden Strahlengängen die Schritte erfolgen:
- Antreiben des zweiten Filterrads zur Rotation um die gemeinsame Achse in einer zweiten, der ersten entgegengesetzten Rotationsrichtung, um den doppelten Verstellwinkel,
- Rückstellen des ersten Filterrads durch Federkraft in die Ausgangsposition des ersten Filterrads,
- Mitnehmen des dritten Filterrads aus der Ausgangsposition des dritten Filterrads zur Rotation in der zweiten Rotationsrichtung um den Verstellwinkel.

Der Verstellwinkel kann insbesondere etwa 90° betragen, wobei in dem ersten, dem zweiten und dem dritten Filterrad vorzugsweise jeweils zwei gleichartige Filter und jeweils zwei freie optische Durchgänge angeordnet sind, wobei die beiden Filter und die beiden freien optischen Durchgänge jeweils einander gegenüber liegen können.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Weitere Aspekte der Erfindung ergeben sich aus der nachfolgenden Beschreibung eines bevorzugten Ausführungsbeispiels und der beigefügten Zeichnung. Es zeigen:
Fig. 1 eine Schnittdarstellung eines Stereo-Operationsmikroskops mit einer Filterwechselvorrichtung gemäß einem Ausführungsbeispiel der Erfindung;
Fig. 2 eine schematische Darstellung der Anordnung der Filter in den drei Filterrädern gemäß dem Ausführungsbeispiel der Erfindung;
Fig. 3 eine schematische Darstellung von drei Filteranordnungen;
Fig. 4 eine schematische Darstellung der Anordnung der Filterräder der Filterwechselvorrichtung, als Draufsicht in Strahlrichtung gesehen;
Fig. 5 die Filterwechselvorrichtung gemäß Fig. 1, in einer Ansicht schräg in Strahlrichtung gesehen;
Fig. 6 die Filterwechselvorrichtung gemäß Fig. 1, in einer Ansicht schräg entgegen der Strahlrichtung gesehen;
Fig. 7 eine Seitenansicht der Filterwechselvorrichtung;
Fig. 8 in einer schematischen Draufsicht drei unterschiedliche Filteranordnungen entsprechend der Darstellung in Fig. 2; und
Fig. 9 in einer schematischen Draufsicht, in Strahlrichtung gesehen, die Filterwechselvorrichtung in zwei Zwischenpositionen der Filterräder.

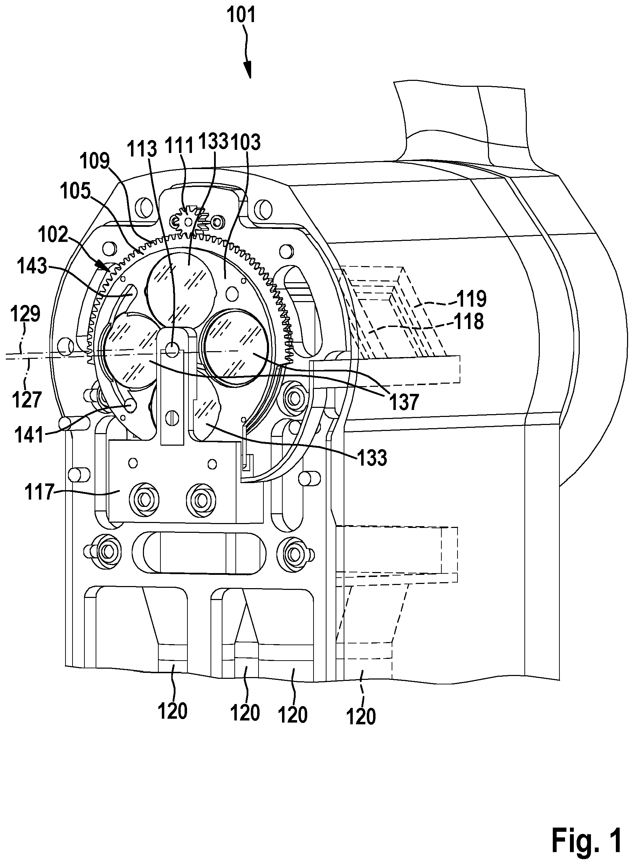

Wie in Fig. 1 beispielhaft dargestellt, umfasst ein Stereo-Operationsmikroskop 101, das zwei Strahlengänge aufweist, eine Filterwechselvorrichtung (Filterwechsler 102). Die beiden Strahlengänge sind die Strahlengänge der beiden Stereokanäle des Stereo-Operationsmikroskops 101 und stellen Beobachtungsstrahlengänge zur stereoskopischen Beobachtung von Gewebe in drei unterschiedlichen Fluoreszenzmodi sowie im Weißlicht dar. In Fig. 1 ist ein Teil des inneren Aufbaus einer drehbaren Optikeinheit des Stereo-Operationsmikroskops gezeigt, ohne eine distalseitig vor der dargestellten Schnittebene angeordnete Zoombaugruppe und ohne das Gehäuse der Optikeinheit. Hinsichtlich des Aufbaus und der Funktion eines geeigneten optischen Beobachtungsinstruments wird auf die am gleichen Tag wie die vorliegende Anmeldung eingereichte deutsche Patentanmeldung desselben Anmelders mit der Bezeichnung "Optisches Beobachtungsinstrument sowie Verfahren zum Erzeugen eines Stereobilds eines Objektfelds" verwiesen.

Der Filterwechsler 102 weist ein erstes Filterrad 103, ein zweites Filterrad 105 und ein da hinter angeordnetes drittes Filterrad 107 auf. Die Filterräder 103, 105 und 107 sind drehbar auf einer feststehenden Rotationsachse 113 gelagert, wobei die Rotationsachse 113 mittels eines Halters 117 endständig befestigt ist. Das erste Filterrad 103, das zweite Filterrad 105 und das dritte Filterrad 107 sind hintereinander angeordnet, wobei alle drei Filterräder 103, 105, 107 jeweils eine mittige Bohrung 139 aufweisen (s.u.), über welche die Filterräder 103, 105 und 107 mit der feststehenden Rotationsachse 113 verbunden sind.

Das erste Filterrad 103 weist in der in Fig. 1 gezeigten Ausgangsposition vertikal gegenüberliegend zwei Filter 133 zur Beobachtung der PPIX- (PDD-) Fluoreszenz (PPIX-Filter 133) und horizontal gegenüberliegend zwei freie optische Durchgänge 137 auf, die jeweils als Öffnungen mit einer ähnlichen Weite wie die Filter 133 ausgebildet sind. Durch die freien optischen Durchgänge des ersten Filterrads 103 sind die zwei Filter des zweiten Filterrads 105 sichtbar (s.u.). Das zweite Filterrad 105 ist halbseitig an einem äußeren Umfang von einem Zahnkranz 109 umgeben, in welchen ein Zahnrad 111 eingreift, das von einem Elektromotor angetrieben wird (s.u.). Des Weiteren weist das zweite Filterrad 105 einen ersten Mitnehmerstift 141 auf, welcher in einen Schlitz 143 des ersten Filterrades 103 eingreift. Die Schlitz 143 erstreckt sich, von der Rotationsachse 113 aus gesehen, näherungsweise über einen Viertelkreis.

Die beiden Strahlengänge des Stereo-Operationsmikroskops sind in der Darstellung in Fig. 1 horizontal gegeneinander versetzt und verlaufen parallel zueinander durch die horizontal stehenden freien optischen Durchgänge 137 des ersten Filterrads 103 in die Zeichenebene hinein. In Fig. 1 sind die optischen Achsen 127, 129 der beiden Strahlengänge angedeutet. Im Bereich des Filterwechslers 102 sind die Strahlengänge afokal. In den Strahlengängen sind nachfolgend jeweils ein dichroitischer Strahlteiler 118 und weiter ein Umlenkspiegel 119 angeordnet, durch die die durch den Filterwechsler 102 hindurchtretende Strahlung spektral aufgeteilt und in Richtung auf Abbildungsoptiken 120 umgelenkt wird, die auf jeweils zwei entsprechend zugeordneten elektronischen Bildaufnehmern ein entsprechendes Bild erzeugen (in Fig. 1 nicht im Einzelnen gezeigt).

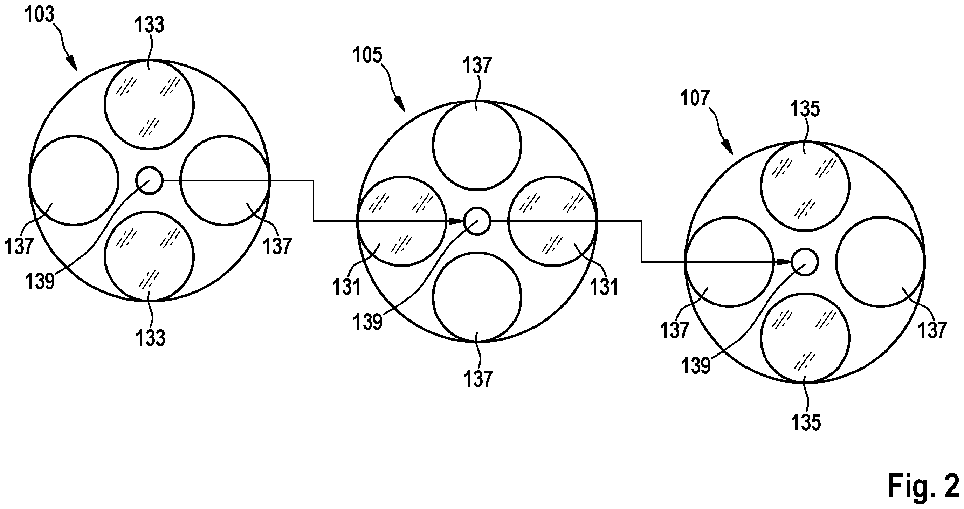

In Fig. 2 ist in schematischer Form die Anordnung der Filter in den drei Filterrädern 103, 105, 107 in einer jeweiligen Ausgangsposition dargestellt. Wie erwähnt, weist das erste Filterrad 103 in der Ausgangsposition vertikal gegenüberliegend zwei PPIX-Filter 133 und horizontal gegenüberliegend zwei freie optische Durchgänge 137 auf. Das zweite Filterrad 105 weist vertikal gegenüberliegend zwei freie optische Durchgänge 137 und horizontal gegenüberliegend zwei Filter 131 zur Beobachtung der ICG-Fluoreszenz (ICG-Filter 131) auf und das dritte Filterrad 107 vertikal gegenüberliegend zwei Filter 135 zur Beobachtung der Fluorescein-Fluoreszenz (Fluorescein-Filter 135) und horizontal gegenüberliegend zwei optische Durchgänge 137.

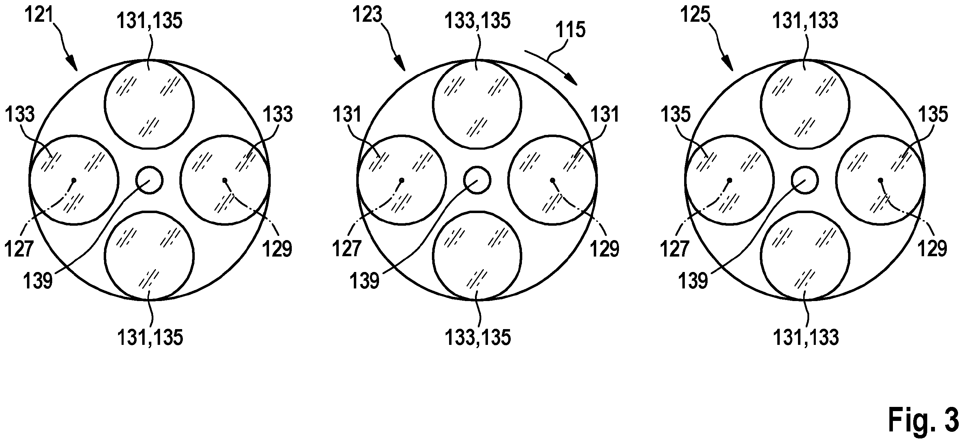

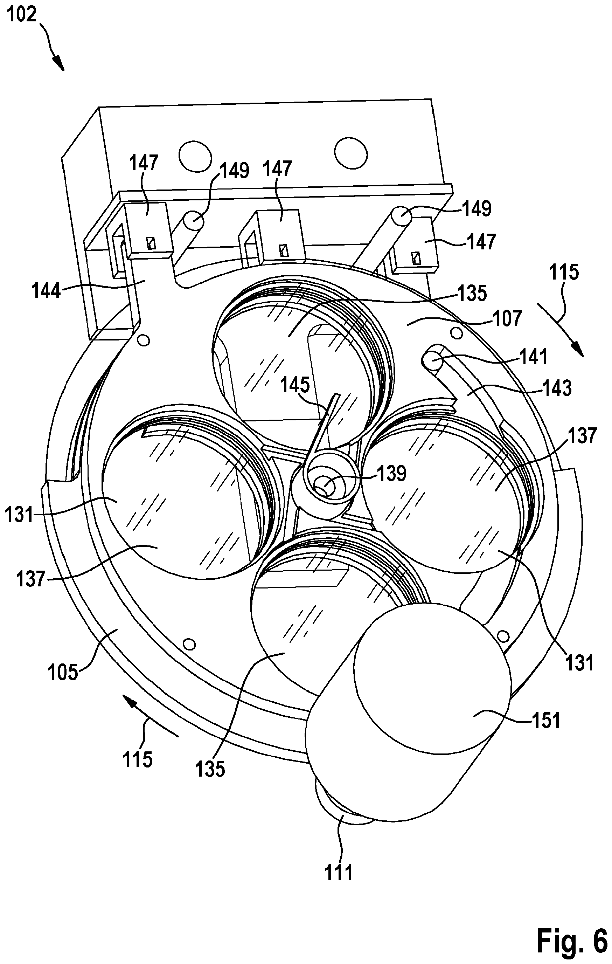

Durch Rotieren des zweiten Filterrades 105 über den Zahnrad-Antrieb mittels des Zahnrads 111, das den Zahnkranz 109 bewegt, in einer Rotationsrichtung 115 des zweiten Filterrades 105 oder entgegen der Rotationsrichtung 115 lassen sich aufgrund der Mitnahme des ersten Filterrades 103 oder des dritten Filterrades 107 über den jeweiligen Mitnehmerstift 141 und ein Stehenlassen des dritten Filterrads 107 bzw. des ersten Filterrads 103 drei Filteranordnungen realisieren. Diese sind in Fig. 3 schematisch gezeigt, in einer Richtung entgegen der Strahlrichtung gesehen, d.h. entsprechend der Ansicht in Fig. 6 bis 9 (s.u.).

In der in Fig. 2 gezeigten Ausgangsposition befinden sich die beiden ICG-Filter 131 des zweiten Filterrades 105 in den beiden Strahlengängen, während das erste und das zweite Filterrad 103, 107 jeweils mit ihren freien optischen Durchgängen 137 in den beiden Stereo-Strahlengängen des Stereo-Operationsmikroskops 101 liegen. Die Filter 131 überdecken näherungsweise jeweils den gesamten Querschnitt des betreffenden Strahlengangs. In Fig. 3 sind die Durchtrittspunkte der optischen Achsen 127, 129 durch die jeweils eingeschwenkten Filter markiert. Entsprechend befinden sich jeweils hintereinanderliegend ein PPIX-Filter 133 des ersten Filterrades 103 und ein Fluorescein-Filter 135 des dritten Filterrades 107 vertikal gegenüberliegend außerhalb der Strahlengänge. Diese Filteranordnung (zweite Filteranordnung 123) ist in der mittleren Abbildung in Fig. 3 dargestellt. Hierdurch sind die durch ICG erzeugte Fluoreszenzstrahlung des beobachteten Gewebebereichs sowie der Gewebebereich im Weißlicht beobachtbar.

Durch eine 90°-Drehung des zweiten Filterrades 105 in der Rotationsrichtung 115 und Mitnahme des ersten Filterrades 103 unter Stehenlassen des dritten Filterrades 107 sind in der ersten Filteranordnung 121, die in der linken Abbildung von Fig. 3 dargestellt ist, jeweils horizontal gegenüberliegend ein PPIX-Filter 133 in dem ersten Stereo-Strahlengang und ein zweites PPIX-Filter 133 in dem zweiten Strahlengang des Stereo-Operationsmikroskops 101 angeordnet. Hierdurch ist die Fluoreszenz von PPIX (PDD-Fluoreszenz) beobachtbar und aufnehmbar, ebenso kann ggf. die Autofluoreszenz des beobachteten Gewebebereichs beobachtbar sein. Vertikal gegenüberliegend stehen hintereinander außerhalb der beiden Strahlengänge jeweils ein ICG-Filter 131 des zweiten Filterrades 105 und ein Fluorescein-Filter 135 des dritten Filterrades 107.

Durch eine Rotation um 90° des zweiten Filterrades 105 entgegen der Rotationsrichtung 115 nach Rückkehr in die Ausgangsposition unter Mitnehmen des dritten Filterrades 107 und unter Stehenlassen des ersten Filterrades 103 wird eine dritte Filteranordnung 125 erreicht (rechte Abbildung von Fig. 3), bei der jeweils ein Fluorescein-Filter 135 des dritten Filterrades 107 horizontal gegenüberliegend in den ersten Strahlengang und in den zweiten Strahlengang eingeschaltet ist, so dass die von Fluorescein emittierte Fluoreszenz beobachtbar ist, während vertikal gegenüberliegend, außerhalb der Strahlengänge jeweils ein ICG-Filter 131 und ein PPIX-Filter 133 hintereinander angeordnet sind.

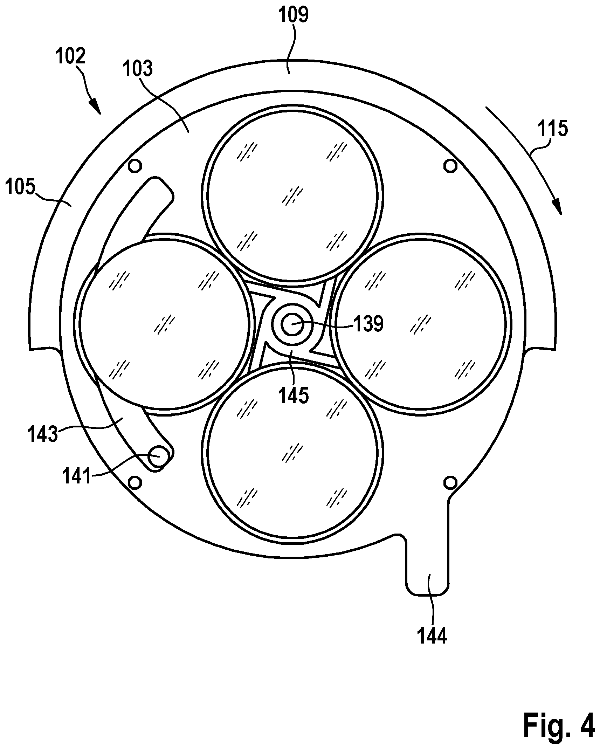

Wie in Fig. 4 in einer axialen Draufsicht, in Strahlrichtung gesehen, auf die näherungsweise kreisförmige Scheibe, die das erste Filterrad 103 bildet, gezeigt ist, trägt das zweite Filterrad 105 an seinem Umfang den etwa um 180° umlaufenden Zahnkranz 109, wobei in Fig. 4 und den nachfolgenden Figuren der Einfachheit halber die Zähne nicht dargestellt sind. An dem halbkreisförmigen Zahnkranz 109 wird das zweite Filterrad 105 über das Zahnrad 111 angetrieben (s.u.). Die Scheibe des ersten Filterrads 103 ist durch den viertelkreisförmigen Schlitz 143 durchbrochen, durch den in Fig. 4 das zweite Filterrad 105 sichtbar ist und in den der erste Mitnehmerstift 141 des zweiten Filterrads 105 eingreift. Des Weiteren weist das erste Filterrad 103 um die mittige Bohrung 139 und somit die feststehende Rotationsachse 113 eine Rückstellfeder 145 zum Rückstellen des ersten Filterrades 103 in die Ausgangsposition auf. Das nicht in Fig. 4 sichtbare dritte Filterrad 107 ist entsprechend zum ersten Filterrad 103 ausgeführt. Des Weiteren weist das erste Filterrad 103 einen Anschlaghebel 144 zum Anschlag an einem Anschlagstift 149 sowie zum Eingreifen in eine Lichtschranke 147 (s.u.) auf. Ebenso wird der halbkreisförmige Zahnkranz 109, der halbseitig den Durchmesser des zweiten Filterrads 105 vergrößert, zur Detektion mittels einer Lichtschranke 147 verwendet.

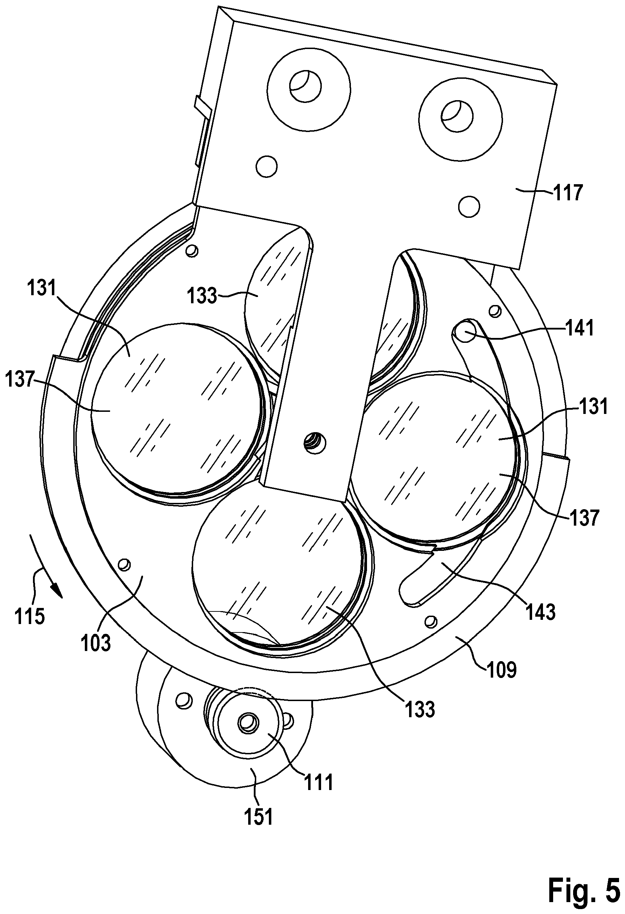

In den Figuren 5 bis 9 ist der Filterwechsler 102 bzw. sind die Filterräder umgekehrt wie in Fig. 1 und Fig. 4 dargestellt, d.h. beispielsweise ist das in Fig. 1 oberhalb des zweiten Filterrads 105 angeordnete Zahnrad 111 in den Figuren 5 bis 9 unterhalb des zweiten Filterrads dargestellt.

In Fig. 5 ist der Filterwechsler 102 in einer Ansicht schräg in Strahlrichtung gesehen dargestellt; diese Ansicht entspricht etwa der Ansicht in Fig. 1, ist jedoch umgekehrt dargestellt.

Wie in Fig. 5 gezeigt, weist das erste Filterrad 103 den viertelkreisförmigen Schlitz 143 auf, in den der erste Mitnehmerstift 141 eingreift. Den Schlitz 143 durchsetzt einer der beiden gegenüber angeordneten freien optischen Durchgänge 137 des ersten Filterrads 103. In dieser Drehstellung, die der ersten Filteranordnung 121 (s. Fig. 3) entspricht, sind vertikal gegenüberliegend und somit außerhalb der Strahlengänge die PPIX-Filter 133 des ersten Filterrads 103 angeordnet. Durch die freien optischen Durchgänge 137 hindurch sind die ICG-Filter 131 des zweiten Filterrads 105 sichtbar.

Weiter ist in Fig. 5 angedeutet, dass der Filterwechsler 102 einen Elektromotor 151 aufweist, welcher mittels des Zahnrads 111 in den Zahnkranz 109 des zweiten Filterrades 105 eingreift, wobei die Zähne nicht dargestellt sind. Dabei wird direkt nur das zweite Filterrad 105 angetrieben, während das erste Filterrad 103 und das dritte Filterrad 107 lediglich über den ersten bzw. zweiten Mitnehmerstift 141 entsprechend der Ausführung der jeweiligen Schlitze 143 mitgenommen werden.

In Fig. 6 ist der Filterwechsler 102 in einer Schrägansicht von hinten (vergleiche Fig. 1), d.h. im Wesentlichen entgegen der Strahlrichtung, dargestellt. Dabei ist das dritte Filterrad 107 sichtbar sowie der Zahnkranz 109 des zweiten Filterrads 105. Das dritte Filterrad 107 weist im Vergleich zum zuvor gezeigten ersten Filterrad 103 entsprechend einen Schlitz 143 auf, in welchen der zweite Mitnehmerstift 141 des zweiten Filterrades 105 eingreift. Das dritte Filterrad 107 weist mittig die Bohrung 139 und eine darum verspannte Rückstellfeder 145 auf. Des Weiteren weist das dritte Filterrad 107 gegenüberliegend jeweils zwei freie optische Durchgänge 137 und zwei Fluorescein-Filter 135 auf, wobei in der in Fig. 6 gezeigten Drehstellung, die der zweiten Filteranordnung 123 (s. Fig. 3) entspricht, vertikal gegenüberliegend und somit außerhalb der Strahlengänge die Fluorescein-Filter 135 angeordnet sind. Durch die optischen Durchgänge 137 hindurch sind die Filter 131 des zweiten Filterrads 105 sichtbar.

Wie in Fig. 6 gezeigt, befindet sich in dieser Drehstellung ein Anschlaghebel 144 des dritten Filterrades 107 im Detektionsbereich der ersten Lichtschranke 147 und schlägt zugleich an den Anschlagstift 149 an. Die mittig angeordnete Lichtschranke 147 dient zur Detektion einer Position des zweiten Filterrades 105 und die rechts in Fig. 5 angeordnete Lichtschranke 147 zur Detektion einer Position des ersten Filterrades 103.

Ausgehend von der in Fig. 5 und 6 gezeigten Ausgangsposition, die der zweiten Filteranordnung 123 (s. Fig. 3) entspricht, wird bei Rotation des zweiten Filterrads 105 in Rotationsrichtung 115 das erste Filterrad 103 durch den ersten Mitnehmerstift 141 mitgenommen (s. Fig. 5), während das dritte Filterrad 107 stehen bleibt, da der zweite Mitnehmerstift 141 sich in dem Schlitz 143 des dritten Filterrads 107 bewegen kann (s. Fig. 5). Durch eine Rotation um 90° wird die erste Filteranordnung 121 (s. Fig. 3) erreicht. Bei einer Rotation des zweiten Filterrads 105 zurück in die Ausgangsposition wird das erste Filterrad 103 durch die Rückstellfeder 145 des ersten Filterrads 103 in seine Ausgangsposition zurückgeführt, wobei ein Anschlaghebel 144 des ersten Filterrads 103 in die Lichtschranke 147 (in Fig. 6 rechts dargestellt) einfährt und zugleich an den entsprechenden Anschlagstift 149 anschlägt.

Umgekehrt wird ausgehend von der zweiten Filteranordnung 123 bei Rotation des zweiten Filterrads 105 entgegen der Rotationsrichtung 115 das dritte Filterrad 107 durch den zweiten Mitnehmerstift 141 mitgenommen (s. Fig. 6), während das erste Filterrad 103 stehen bleibt, da der erste Mitnehmerstift 141 sich in dem Schlitz 143 des dritten Filterrads 103 bewegen kann (s. Fig. 5). Hierdurch wird die dritte Filteranordnung 125 (s. Fig. 3) erreicht. Anschließend kann das dritte Filterrad 107 mittels der Rückstellfeder 145 wieder in seine Ausgangsposition zurückgestellt werden, wobei der Anschlaghebel 144 des dritten Filterrads 107 in die Lichtschranke 147 (in Fig. 6 links dargestellt) einfährt und zugleich an den entsprechenden Anschlagstift 149 anschlägt.

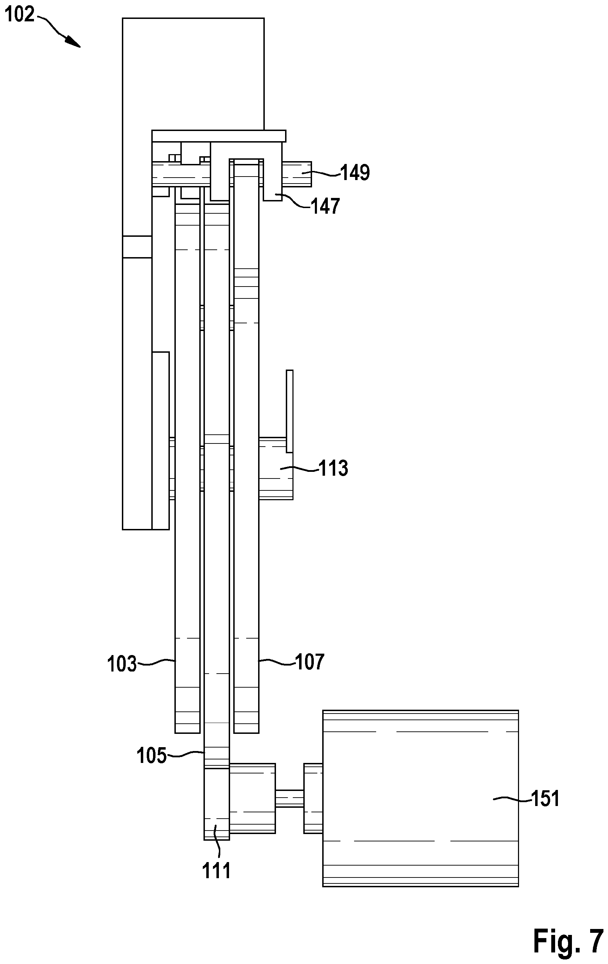

In Fig. 7 ist der Filterwechsler 102 in einer Seitenansicht dargestellt. Wie in Fig. 7 zu erkennen ist, sind die Filterräder 103, 105, 107 mit geringem Abstand zueinander angeordnet, so dass der Filterwechsler 102 insgesamt nur einen geringfügig größeren Raumbedarf als ein einzelnes der Filterräder 103, 105, 107 hat und somit sehr kompakt ausgeführt werden kann.

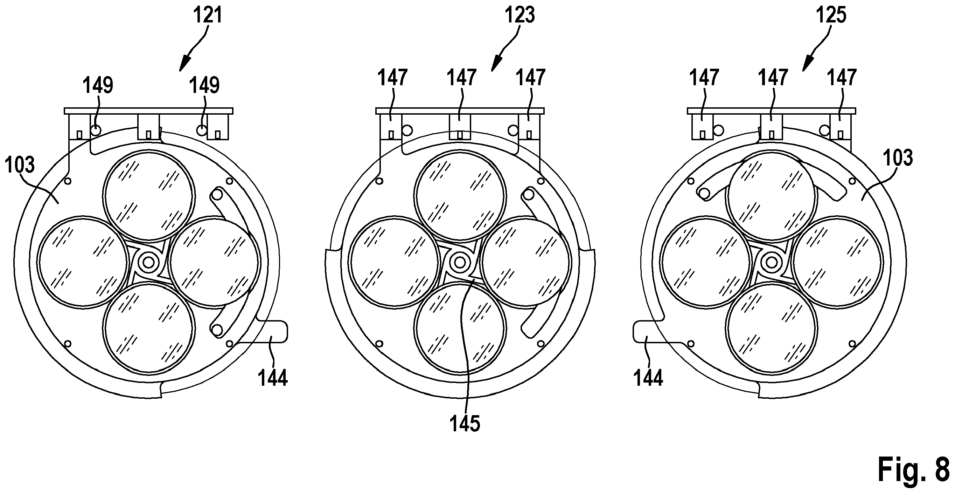

In Fig. 8 sind drei verschiedene Filteranordnungen gezeigt, die den Filteranordnungen 121, 123, 125 in Fig. 3 entsprechen, ebenfalls entgegen der Strahlrichtung gesehen. Dabei ist zu erkennen, dass in der zweiten Filteranordnung 123, die die Ausgangsposition darstellt, die linke und die rechte Lichtschranke 147 unterbrochen sind. In der ersten Filteranordnung 121 sind die linke und die mittlere Lichtschranke 147 unterbrochen, während in der dritten Filteranordnung 125 die mittlere und die rechte Lichtschranke 147 unterbrochen sind. Aus den Signalen der Lichtschranken 147 lässt sich somit eine jeweilige Filterposition ermitteln.

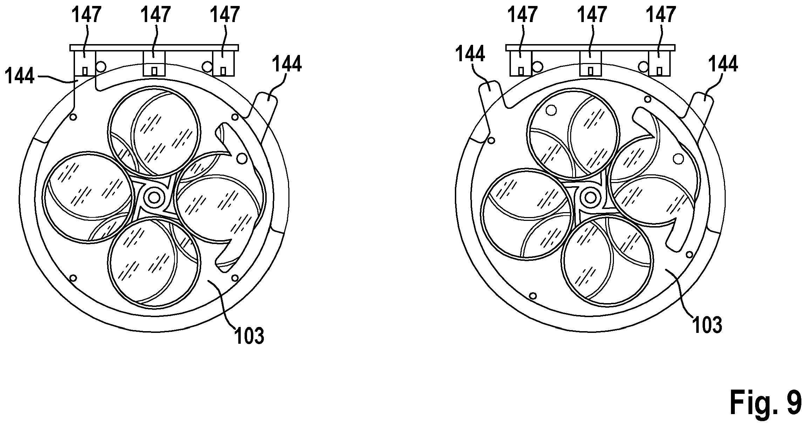

Ferner ist, wie in Fig. 9 in der linken Abbildung gezeigt ist, in solchen Fällen, in den nur die linke (oder entsprechend nur die rechte) Lichtschranke 147 unterbrochen ist, davon auszugehen, dass das zweite Filterrad 103 noch in der Bewegung ist und eine entsprechende Endstellung noch nicht erreicht ist. Gemäß der rechten Abbildung in Fig. 9 ist andererseits dann, wenn keine der Lichtschranken unterbrochen ist, von einem Fehlerzustand auszugehen, bei dem beispielsweise das erste oder das dritte Filterrad 103, 107 verklemmt ist und durch die jeweilige Rückstellfeder 145 nicht mehr in seine Ausgangsposition zurückgestellt werden kann. Aus den Signalen der Lichtschranken 147 lässt sich somit auch darauf schließen, ob eine Rotationsbewegung noch nicht abgeschlossen ist, sowie ggf. auf einen Fehlerzustand des Filterwechslers 102.

Somit ist ein Stereo-Operationsmikroskop 101 mit einem sehr kompakten Filterwechsler 102 realisiert, welcher einen sehr schnellen Wechsel zwischen ICG-Filter 131, PPIX-Filter 133 und Fluorescein-Filter 135 und somit zwischen drei Fluoreszenzmodi realisiert und folglich eine optimale Differenzierung eines zu untersuchenden Gewebes ermöglicht.

Der Übersichtlichkeit halber sind nicht in allen Figuren alle Bezugszeichen dargestellt. Zu einer Figur nicht erläuterte Bezugszeichen haben die gleiche Bedeutung wie in den übrigen Figuren.

### Bezugszeichenliste

- 101: Stereo-Operationsmikroskop
- 102: Filterwechsler
- 103: Erstes Filterrad
- 105: Zweites Filterrad
- 107: Drittes Filterrad
- 109: Zahnkranz
- 111: Zahnrad
- 113: Rotationsachse
- 115: Rotationsrichtung
- 117: Halter
- 118: Strahlteiler
- 119: Umlenkspiegel
- 120: Abbildungsoptik
- 121: Erste Filteranordnung
- 123: Zweite Filteranordnung
- 125: Dritte Filteranordnung
- 127: Optische Achse
- 129: Optische Achse
- 131: ICG-Filter
- 133: PPIX-Filter
- 135: Fluorescein-Filter
- 137: Freier optischer Durchgang
- 139: Bohrung
- 141: Mitnehmerstift
- 143: Schlitz
- 144: Anschlaghebel
- 145: Rückstellfeder
- 147: Lichtschranke
- 149: Anschlagstift
- 151: Elektromotor

## Patentansprüche

1. Filterwechselvorrichtung für ein optisches Beobachtungsinstrument mit zwei Strahlengängen, insbesondere für ein stereoskopisches Beobachtungsinstrument, insbesondere für ein Stereo-Videoendoskop, ein Stereo-Exoskop oder ein Stereo-Operationsmikroskop (101), wobei die Filterwechselvorrichtung ein erstes Filterrad (103), ein zweites Filterrad (105) und ein drittes Filterrad (107) aufweist, wobei die Filterräder (103, 105, 107) entlang einer gemeinsamen Achse hintereinander angeordnet und um die gemeinsame Achse und zueinander rotierbar sind, wobei jedes Filterrad (103, 105, 107) mindestens einen Filter (131, 133, 135) und mindestens einen freien optischen Durchgang (137) aufweist, so dass in jeden der beiden Strahlengänge ein Filter (131, 133, 135) oder ein freier optischer Durchgang (137) eines jeden Filterrads (103, 105, 107) einbringbar ist, und wobei das zweite Filterrad (105) antreibbar ist und das erste Filterrad (103) über einen ersten Mitnehmer und das dritte Filterrad (107) über einen zweiten Mitnehmer mit dem zweiten Filterrad (105) gekoppelt sind.

2. Filterwechselvorrichtung nach Anspruch 1, **dadurch gekennzeichnet, dass** das zweite Filterrad (105) zwischen dem ersten Filterrad (103) und dem dritten Filterrad (107) angeordnet ist.

3. Filterwechselvorrichtung nach Anspruch 1 oder 2, **dadurch gekennzeichnet, dass** das erste Filterrad (103) über den ersten Mitnehmer derart mit dem zweiten Filterrad (105) gekoppelt ist, dass ausgehend von einer Ausgangsposition das erste Filterrad (103) bei einer Rotation des zweiten Filterrads (105) in einer ersten Rotationsrichtung (115) mitgenommen und in einer zweiten Rotationsrichtung stehengelassen wird.

4. Filterwechselvorrichtung nach einem der vorhergehenden Ansprüche, **dadurch gekennzeichnet, dass** das dritte Filterrad (107) über den zweiten Mitnehmer derart mit dem zweiten Filterrad (105) gekoppelt ist, dass ausgehend von einer Ausgangsposition das dritte Filterrad (107) bei einer Rotation des zweiten Filterrads in einer ersten Rotationsrichtung (115) stehengelassen und in einer zweiten Rotationsrichtung mitgenommen wird.

5. Filterwechselvorrichtung nach Anspruch 3 oder 4, **dadurch gekennzeichnet, dass** der erste und/oder der zweite Mitnehmer als Mitnehmerstift (141) des zweiten Filterrads (105) ausgebildet ist, welcher in eine Aussparung des ersten Filterrades (103) bzw. eine Aussparung des dritten Filterrades (107) eingreift und in der der Mitnehmerstift (141) in einem Freilaufwinkelbereich freilaufbar ist.

6. Filterwechselvorrichtung nach Anspruch 5, **dadurch gekennzeichnet, dass** die Aussparung des ersten Filterrads (103) und/oder des dritten Filterrads (107) als ringsegmentförmige Nut oder als ringsegmentförmiger Schlitz (143) ausgebildet ist.

7. Filterwechselvorrichtung nach einem der vorhergehenden Ansprüche, **dadurch gekennzeichnet, dass** das erste Filterrad (103) und/oder das dritte Filterrad (107) eine Rückstelleinrichtung, insbesondere eine Rückstellfeder (145), zum Rückstellen des jeweiligen Filterrades in eine Ausgangsposition aufweist oder aufweisen.

8. Filterwechselvorrichtung nach einem der vorhergehenden Ansprüche, **dadurch gekennzeichnet, dass** das erste Filterrad (103) zwei Filter mit einer ersten Filtercharakteristik und zwei freie optische Durchgänge (137) aufweist, das zweite Filterrad (105) zwei Filter mit einer zweiten Filtercharakteristik und zwei freie optische Durchgänge (137) und/oder das dritte Filterrad (107) zwei Filter mit einer dritten Filtercharakteristik und zwei freie optische Durchgänge (137) aufweist oder aufweisen.

9. Filterwechselvorrichtung nach einem der vorhergehenden Ansprüche, **dadurch gekennzeichnet, dass** das erste Filterrad (103) jeweils einander gegenüberliegend zwei Filter mit einer ersten Filtercharakteristik und zwei freie optische Durchgänge (137) aufweist, das zweite Filterrad (105) jeweils einander gegenüberliegend zwei Filter mit einer zweiten Filtercharakteristik und zwei freie optische Durchgänge (137) und/oder das dritte Filterrad (107) jeweils einander gegenüberliegend zwei Filter mit einer dritten Filtercharakteristik und zwei freie optische Durchgänge (137) aufweist oder aufweisen.

10. Filterwechselvorrichtung nach einem der vorhergehenden Ansprüche, **dadurch gekennzeichnet, dass** mindestens eines der Filter ein Fluoreszenzfilter ist, insbesondere dass die Filter mit der ersten, zweiten und/oder dritten Filtercharakteristik Fluoreszenzfilter sind.

11. Filterwechselvorrichtung nach einem der vorhergehenden Ansprüche, **dadurch gekennzeichnet, dass** das zweite Filterrad (105) motorisch antreibbar ist.

12. Filterwechselvorrichtung nach einem der vorhergehenden Ansprüche, **dadurch gekennzeichnet, dass** das zweite Filterrad (105) an seinem äußeren Umfang antreibbar ist, insbesondere über einen an seinem äußeren Umfang angeordneten Zahnkranz (109), in den ein Zahnrad (111) eingreift.

13. Filterwechselvorrichtung nach einem der vorhergehenden Ansprüche, **dadurch gekennzeichnet, dass** die Filterwechselvorrichtung eine Positionserkennungseinrichtung zum Bestimmen einer jeweiligen Drehposition mindestens eines der Filterräder (103, 105, 107) aufweist.

14. Filterwechselvorrichtung nach Anspruch 13, **dadurch gekennzeichnet, dass** die Positionserkennungseinrichtung mindestens eine Lichtschranke (147) aufweist und mindestens eines der Filterräder (103, 105, 107) eine zugehörige Fahne aufweist.

15. Filterwechselvorrichtung nach einem der vorhergehenden Ansprüche, **dadurch gekennzeichnet, dass** die Filterwechselvorrichtung eine mechanische Anschlagseinrichtung oder mehrere mechanische Anschlagseinrichtungen zur Begrenzung einer Rotation des ersten Filterrads (103), des zweiten Filterrads (105) und/oder des dritten Filterrads (107) aufweist.

16. Optisches Beobachtungsinstrument mit zwei Strahlengängen, insbesondere stereoskopisches Beobachtungsinstrument, insbesondere Stereo-Videoendoskop, Stereo-Exoskop oder Stereo-Operationsmikroskop (101), **dadurch gekennzeichnet, dass** das optische Beobachtungsinstrument eine Filterwechselvorrichtung nach einem der Ansprüche 1 bis 15 aufweist.

17. Verfahren zum Wechseln eines Filters eines optischen Beobachtungsinstruments, das zwei Strahlengänge aufweist, insbesondere eines stereoskopischen Beobachtungsinstruments, insbesondere eines Stereo-Videoendoskops, eines Stereo-Exoskops oder eines Stereo-Operationsmikroskops (101), wobei das optische Beobachtungsinstrument eine Filterwechselvorrichtung nach einem der Ansprüche 1 bis 15 aufweist, wobei für einen ersten Filterwechsel
- das zweite Filterrad (105) aus einer Ausgangsposition des zweiten Filterrads (105) zur Rotation um die gemeinsame Achse in einer ersten Rotationsrichtung (115) um einen Verstellwinkel angetrieben wird,
- das erste Filterrad (103) aus einer Ausgangsposition des ersten Filterrads (103) mittels des ersten Mitnehmers zur Rotation in der ersten Rotationsrichtung (115) um den Verstellwinkel mitgenommen wird, und
- das dritte Filterrad (107) in einer Ausgangsposition des dritten Filterrads (107) stehen gelassen wird,
und für einen weiteren Filterwechsel
- das zweite Filterrad (105) zur Rotation um die gemeinsame Achse in einer zweiten, der ersten entgegengesetzten Rotationsrichtung, um den doppelten Verstellwinkel angetrieben wird,
- das erste Filterrad (103) durch Federkraft in die Ausgangsposition des ersten Filterrads (103) zurückgestellt wird, und
- das dritte Filterrad (107) aus der Ausgangsposition des dritten Filterrads (107) zur Rotation in der zweiten Rotationsrichtung um den Verstellwinkel mitgenommen wird.

## Claims

1. A filter interchange apparatus for an optical observation instrument having two beam paths, in particular for a stereoscopic observation instrument, in particular for a stereo video endoscope, a stereo exoscope or a stereo surgical microscope (101), wherein the filter interchange apparatus has a first filter wheel (103), a second filter wheel (105) and a third filter wheel (107), wherein the filter wheels (103, 105, 107) are arranged in succession along a common axle and are rotatable about the common axle and relative to one another, wherein each filter wheel (103, 105, 107) has at least one filter (131, 133, 135) and at least one free optical passage (137) such that a filter (131, 133, 135) or a free optical passage (137) of each filter wheel (103, 105, 107) is introducible into each of the two beam paths and wherein the second filter wheel (105) is drivable and the first filter wheel (103) is coupled to the second filter wheel (105) via a first entrainment element and the third filter wheel (107) is coupled to the second filter wheel (105) via a second entrainment element.

2. The filter interchange apparatus according to claim 1, **characterized in that** the second filter wheel (105) is arranged between the first filter wheel (103) and the third filter wheel (107).

3. The filter interchange apparatus according to claim 1 or 2, **characterized in that** the first filter wheel (103) is coupled to the second filter wheel (105) via the first entrainment element in such manner that, proceeding from an initial position, the first filter wheel (103) is entrained in the case of a rotation of the second filter wheel (105) in a first direction of rotation (115) and left standing in a second direction of rotation.

4. The filter interchange apparatus according to one of the preceding claims, **characterized in that** the third filter wheel (107) is coupled to the second filter wheel (105) via the second entrainment element in such manner that, proceeding from an initial position, the third filter wheel (107) is left standing in the case of a rotation of the second filter wheel in a first direction of rotation (115) and entrained in a second direction of rotation.

5. The filter interchange apparatus according to claim 3 or 4, **characterized in that** the first and/or the second entrainment element is designed as an entrainment pin (141) of the second filter wheel (105), which engages into a cutout of the first filter wheel (103) or a cutout of the third filter wheel (107) and in which cutout the entrainment pin (141) is able to freewheel within a freewheel angle range.

6. The filter interchange apparatus according to claim 5, **characterized in that** the cutout of the first filter wheel (103) and/or of the third filter wheel (107) is designed as a ring segment-shaped groove or as a ring segment-shaped slot (143).

7. The filter interchange apparatus according to one of the preceding claims, **characterized in that** the first filter wheel (103) and/or the third filter wheel (107) has or have a restoring device, in particular a restoring spring (145), for restoring the respective filter wheel to an initial position.

8. The filter interchange apparatus according to one of the preceding claims, **characterized in that** the first filter wheel (103) has two filters with a first filter characteristic and two free optical passages (137), the second filter wheel (105) has two filters with a second filter characteristic and two free optical passages (137) and/or the third filter wheel (107) has two filters with a third filter characteristic and two free optical passages (137).

9. The filter interchange apparatus according to one of the preceding claims, **characterized in that** the first filter wheel (103) has two filters with a first filter characteristic lying opposite one another and two free optical passages (137) lying opposite one another, the second filter wheel (105) has two filters with a second filter characteristic lying opposite one another and two free optical passages (137) lying opposite one another and/or the third filter wheel (107) has two filters with a third filter characteristic lying opposite one another and two free optical passages (137) lying opposite one another.

10. The filter interchange apparatus according to one of the preceding claims, **characterized in that** at least one of the filters is a fluorescence filter, in particular **in that** the filters with the first, second and/or third filter characteristic are fluorescence filters.

11. The filter interchange apparatus according to one of the preceding claims, **characterized in that** the second filter wheel (105) is drivable by a motor.

12. The filter interchange apparatus according to one of the preceding claims, **characterized in that** the second filter wheel (105) is drivable at its outer circumference, in particular by way of a gear rim (109) which is arranged on its outer circumference and which meshes with a pinion (111).

13. The filter interchange apparatus according to one of the preceding claims, **characterized in that** the filter interchange apparatus has a position identifying device for determining a respective rotational position of at least one of the filter wheels (103, 105, 107).

14. The filter interchange apparatus according to claim 13, **characterized in that** the position identifying device has at least one photoelectric sensor (147) and at least one of the filter wheels (103, 105, 107) has an associated flag.

15. The filter interchange apparatus according to one of the preceding claims, **characterized in that** the filter interchange apparatus has a mechanical stop device or a plurality of mechanical stop devices for restricting a rotation of the first filter wheel (103), of the second filter wheel (105) and/or of the third filter wheel (107).

16. An optical observation instrument having two beam paths, in particular a stereoscopic observation instrument, in particular a stereo video endoscope, stereo exoscope or stereo surgical microscope (101), **characterized in that** the optical observation instrument has a filter interchange apparatus according to one of claims 1 to 15.

17. A method for changing a filter of an optical observation instrument having two beam paths, in particular of a stereoscopic observation instrument, in particular of a stereo video endoscope, a stereo exoscope or a stereo surgical microscope (101), wherein the optical observation instrument has a filter interchange apparatus according to one of claims 1 to 15, wherein, for a first filter change,
- the second filter wheel (105) is driven through an adjustment angle in a first direction of rotation (115) from an initial position of the second filter wheel (105) for rotation about the common axle,
- the first filter wheel (103) is entrained through the adjustment angle from an initial position of the first filter wheel (103) by means of the first entrainment element for rotation in the first direction of rotation (115), and
- the third filter wheel (107) is left standing in an initial position of the third filter wheel (107),
and, for a further filter change,
- the second filter wheel (105) is driven through twice the adjustment angle in a second direction of rotation, opposite to the first, for rotation about the common axle,
- the first filter wheel (103) is restored to the initial position of the first filter wheel (103) by spring force and
- the third filter wheel (107) is entrained through the adjustment angle from the initial position of the third filter wheel (107) for rotation in the second direction of rotation.

## Revendications

1. Appareil d'échange de filtres pour un instrument optique d'observation à deux chemins de faisceau, en particulier pour un instrument d'observation stéréoscopique, en particulier pour un endoscope vidéo stéréo, un exoscope stéréo ou un microscope chirurgical stéréo (101), dans lequel l'appareil d'échange de filtres comporte une première roue à filtres (103), une deuxième roue à filtres (105) et une troisième roue à filtres (107), dans lequel les roues à filtre (103, 105, 107) sont disposées successivement le long d'un axe commun et peuvent tourner autour de l'axe commun et l'une par rapport à l'autre, dans lequel chaque roue à filtres (103, 105, 107) comporte au moins un filtre (131, 133, 135) et au moins un passage optique libre (137) de tel sorte qu'un filtre (131, 133, 135) ou un passage optique libre ( 137) de chaque roue à filtres (103, 105, 107) peut être introduit dans chacun des deux chemins de faisceau et dans lequel la deuxième roue à filtres (105) peut être entraînée et la première roue à filtres (103) est couplée à la deuxième roue à filtres (105 ) par l'intermédiaire d'un premier élément d'entraînement et la troisième roue à filtres (107) est couplée à la deuxième roue à filtres (105) par l'intermédiaire d'un second élément d'entraînement.

2. Appareil d'échange de filtres selon la revendication 1, **caractérisé en ce que** la deuxième roue à filtres (105) est disposée entre la première roue à filtres (103) et la troisième roue à filtres (107).

3. Appareil d'échange de filtres selon la revendication 1 ou 2, **caractérisé en ce que** la première roue à filtres (103) est couplée à la deuxième roue à filtres (105) par l'intermédiaire du premier élément d'entraînement de telle manière que, en partant d'une position initiale, la première roue à filtres (103) est entraînée en cas de rotation de la deuxième roue à filtres (105) dans un premier sens de rotation (115) et laissée à l'arrêt dans un deuxième sens de rotation.

4. Appareil d'échange de filtres selon l'une des revendications précédentes, **caractérisé en ce que** la troisième roue à filtres (107) est couplée à la deuxième roue à filtres (105) par l'intermédiaire du second élément d'entraînement de telle manière que, en partant d'une position initiale, la troisième roue à filtres (107) est laissée à l'arrêt en cas de rotation de la deuxième roue à filtres dans un premier sens de rotation (115) et entraînée dans un deuxième sens de rotation.

5. Appareil d'échange de filtres selon la revendication 3 ou 4, **caractérisé en ce que** le premier et/ou le second élément d'entraînement est conçu comme une tige d'entraînement (141) de la deuxième roue à filtres (105), qui s'engage dans une découpe de la première roue à filtres (103) ou une découpe de la troisième roue à filtres (107) et dans laquelle découpe la tige d'entraînement (141) peut tourner en roue libre dans une plage d'angle de roue libre.

6. Appareil d'échange de filtres selon la revendication 5, **caractérisé en ce que** la découpe de la première roue à filtres (103) et/ou de la troisième roue à filtres (107) est conçue comme une rainure en forme de segment annulaire ou d'une fente en forme de segment annulaire (143).

7. Appareil d'échange de filtres selon l'une des revendications précédentes, **caractérisé en ce que** la première roue à filtres (103) et/ou la troisième roue à filtres (107) comporte ou comportent un dispositif de rappel, en particulier un ressort de rappel (145), pour rappeler la roue à filtres respective dans une position initiale.

8. Appareil d'échange de filtres selon l'une des revendications précédentes, **caractérisé en ce que** la première roue à filtres (103) comporte deux filtres avec une première caractéristique de filtrage et deux passages optiques libres (137), la deuxième roue à filtres (105) comporte deux filtres avec une deuxième caractéristique de filtrage et deux passages optiques libres (137) et/ou la troisième roue à filtres (107) comporte deux filtres avec une troisième caractéristique de filtrage et deux passages optiques libres (137).

9. Appareil d'échange de filtres selon l'une des revendications précédentes, **caractérisé en ce que** la première roue à filtres (103) comporte deux filtres avec une première caractéristique de filtre se faisant face et deux passages optiques libres (137) se faisant face, la deuxième roue à filtres (105) comporte deux filtres avec une deuxième caractéristique de filtre se faisant face et deux passages optiques libres (137) se faisant face et/ou la troisième roue à filtres (107) comporte deux filtres avec une troisième caractéristique de filtre se faisant face et deux passages optiques libres (137) se faisant face.

10. Appareil d'échange de filtres selon l'une des revendications précédentes, **caractérisé en ce qu'au** moins un des filtres est un filtre à fluorescence, en particulier **en ce que** les filtres avec la première, la deuxième et/ou la troisième caractéristiques de filtre sont des filtres à fluorescence.

11. Appareil d'échange de filtres selon l'une des revendications précédentes, **caractérisé en ce que** la deuxième roue à filtres (105) peut être entraînée par un moteur.

12. Appareil d'échange de filtres selon l'une des revendications précédentes, **caractérisé en ce que** la deuxième roue à filtres (105) peut être entraînée sur sa circonférence extérieure, en particulier par l'intermédiaire d'une couronne dentée (109) qui est disposée sur sa circonférence extérieure et qui s'engage avec un pignon (111).

13. Appareil d'échange de filtres selon l'une des revendications précédentes, **caractérisé en ce que** l'appareil d'échange de filtres comporte un dispositif d'identification de position pour déterminer une position de rotation respective d'au moins une des roues à filtres (103, 105, 107).

14. Appareil d'échange de filtres selon la revendication 13, **caractérisé en ce que** le dispositif d'identification de position comporte au moins un capteur photoélectrique (147) et au moins l'une des roues à filtres (103, 105, 107) comporte un indicateur associé.

15. Appareil d'échange de filtres selon l'une des revendications précédentes, **caractérisé en ce que** l'appareil d'échange de filtre comporte un dispositif d'arrêt mécanique ou une pluralité de dispositifs d'arrêt mécanique pour limiter une rotation de la première roue de filtre (103), de la deuxième roue à filtres (105) et/ou de la troisième roue à filtres (107).

16. Instrument d'observation optique à deux chemins de faisceau, en particulier un instrument d'observation stéréoscopique, en particulier un endoscope vidéo stéréo, un exoscope stéréo ou un microscope chirurgical stéréo (101), **caractérisé en ce que** l'instrument d'observation optique comporte un dispositif d'échange de filtres selon l'une des revendications 1 à 15.

17. Procédé pour changer un filtre d'un instrument d'observation optique à deux chemins de faisceau, en particulier d'un instrument d'observation stéréoscopique, en particulier d'un endoscope vidéo stéréo, un exoscope stéréo ou un microscope chirurgical stéréo (101), dans lequel l'instrument d'observation optique comporte un dispositif d'échange de filtres selon l'une des revendications 1 à 15, dans lequel, pour un premier changement de filtre,
- la deuxième roue à filtres (105) est entraînée sur un angle de réglage dans un premier sens de rotation (115) depuis une position initiale de la deuxième roue à filtres (105) pour une rotation autour de l'axe commun,
- la première roue à filtres (103) est entraînée sur l'angle de réglage depuis une position initiale de la première roue à filtres (103) au moyen du premier élément d'entraînement pour une rotation dans le premier sens de rotation (115), et
- la troisième roue à filtres (107) est laissée à l'arrêt dans une position initiale de la troisième roue à filtres (107),
et, pour un autre changement de filtre,
- la deuxième roue à filtres (105) est entraînée sur deux fois l'angle de réglage dans un deuxième sens de rotation, faisant face au premier, pour la rotation autour de l'axe commun,
- la première roue à filtres (103) est ramenée à la position initiale de la première roue à filtres (103) par la force d'un ressort et
- la troisième roue à filtres (107) est entraînée sur l'angle de réglage depuis la position initiale de la troisième roue à filtres (107) pour une rotation dans le deuxième sens de rotation.
